# EUROPEAN PATENT APPLICATION

(11) **EP 3 477 608 A1**
(43) Date of publication of application: **01.05.2019**
(21) Application number: 17815221.1
(22) Date of filing: 12.06.2017
(51) Int. Cl.: G08B 25/04, A61G 12/00, G08B 21/02

(54) **CENTRAL PROCESSING DEVICE FOR MONITORED-PERSON MONITORING SYSTEM, CENTRAL PROCESSING METHOD, AND MONITORED-PERSON MONITORING SYSTEM**

(30) Priority: 24.06.2016 JP 2016126041
(71) Applicant: Konica Minolta, Inc., Tokyo 100-7015 (JP)
(72) Inventor: NISHIKADO, Masashi, Tokyo 100-7015 (JP); UESAKA, Takeshi, Tokyo 100-7015 (JP); KOGO, Shoji, Tokyo 100-7015 (JP); SAKAKIBARA, Satoru, Tokyo 100-7015 (JP)
(74) Representative: Hoffmann Eitle
(86) International application number: PCT/JP2017/021565
(87) International publication number: WO 2017/221752

(57) **Abstract**

In this central processing device and method, an event received from a sensor device provided in correspondence with a monitored person is stored as monitoring information associated with the monitored person, wherein the monitoring information includes a detection time or a reception time of the event and an event type. With the central processing device and the method, a type of an event corresponding to a predetermined monitored person in a predetermined period is extracted from the stored monitoring information, and the events in the predetermined period are displayed on a predetermined display device by type and in association with the monitored person on the basis of the extraction result. The monitored-person monitoring system according to the present invention includes the central processing device.

## Description

### Technical Field

The present invention relates to a central processing device for a monitored-person monitoring system for monitoring a monitored person to be monitored by using a plurality of devices, a central processing method, and the monitored-person monitoring system.

### Background Art

In Japan, according to improvement in the standard of living with high economic growth after the war, improvement in hygienic environment and medical level, Japan has an aging population. More specifically, Japan has a super-aging society in which a population aging rate which is a rate of the population aged 65 and over relative to a total population exceeds 21%. According to the statistics made in September 2013, the aged population is about 31.86 million, and the population aged rate is 25.0%, and one in four people is an aged person. In 2035, it is predicted that the aged population becomes about 37.41 million and that one in three people is an aged person (Statistics Bureau, Ministry of Internal Affairs and Communications, Japan). In such an aged society, it is expected that an increase in people who need nursing and care (person who needs nursing care) due to illness, injury, an advanced age, and the like is larger than that in a normal society which is not the aged society. In addition, Japan is a society with a low birth rate, for example, in which the total fertility rate is 1.43 in 2013. For that reason, "elderly care by the elderly" occurs in which an aged family member (spouse, child, brother and sister) gives nursing care to an aged person who needs nursing care.

The person who needs nursing care moves to facilities such as a hospital, a welfare facility for the aged (short-term admission facility for the elderly, elderly nursing home, special elderly nursing home under laws of Japan) and receives nursing and nursing care. In such a facility, a case may occur in which the person who needs nursing care gets injured, for example, due to falling from the bed and falling down at the time of walking and gets out of the bed and wanders around. It is necessary to deal with such a situation as soon as possible. Furthermore, if this situation is left alone, this may cause a serious situation. Therefore, in the facility, nurses and caregivers (nursing caregiver) regularly go around to check safety and a condition of the person who needs nursing care.

However, an increase in the number of nursing caregivers does not catch up with an increase in the number of people who need nursing care, and the nursing and nursing care industries are chronically in an understaffing situation. In addition, since the number of nursing caregivers in a time of semi-night shift and night shift is smaller than that in day shift, a work load for one person increases, and it is required to reduce the work load. Furthermore, the facility is no exception in that the situation of the "elderly care by the elderly" occurs, an aged nursing caregiver often cares aged person who needs nursing care. Since the physical strength is weakened as getting order, a load of care for the aged caregiver becomes heavier than that for a young care person even if the aged caregiver has normal health. In addition, movement and judgment will be slower.

To solve the shortage of labor and reduce the load of the nursing caregiver, a technique has been required which supplement a nursing work and a nursing care work. Therefore, in recent years, a monitored-person monitoring technique for monitoring (monitoring) a monitored person to be monitored who is a person who needs nursing care has been researched and developed.

One of the techniques is, for example, a nurse call system disclosed in Patent Literature 1. The nurse call system disclosed in Patent Literature 1 includes a nurse call handset which is provided at the bed and is used to call a nurse by a patient and a nurse call master unit which is provided at a nurse station and is used to respond to the call from the nurse call handset. The nurse call system includes a camera which images the patient on the bed from above the bed and a state determination unit which determines occurrence of at least one of a state where the patient sits up and a state where the patient leaves the bed on the basis of the image imaged by the camera and outputs a caution state occurrence signal, and the nurse call master unit includes a notification unit which performs a notification operation in response to the caution state occurrence signal. The nurse call system includes a mobile terminal which is carried by the nurse to respond to the call from the nurse call handset and a communication control unit which receives the caution state occurrence signal and transmits the image imaged by the camera to the mobile terminal.

On the other hand, from the viewpoint of confirming safety, a person who lives alone is similar to the person who needs nursing and is a monitoring target.

By the way, a degree of weakness of physical ability, a degree and place of injury, a degree and place of paralysis, a life rhythm in the past (life habit), how to use the nurse call, and the like vary depending on individual monitored person who is a person who needs nursing care. Therefore, for example, how to issue a notification of the caution state occurrence signal and how to issue a notification of the nurse call vary depending on individual monitored person. When a predetermined tendency in the way of notification depending on each of the monitored persons is found, it is possible to provide more appropriate nursing and nursing care according to the predetermined tendency.

The nurse call system disclosed in Patent Literature 1 can issue a notification of the caution state occurrence signal and the nurse call. However, the nurse call system only executes the notification, and Patent Literature 1 does not disclose the description regarding the tendency in how to issue a notification.

### Citation List

### Patent Literature

Patent Literature 1: JP 2014-90913 A

### Summary of Invention

The present invention has been made in view of the above circumstances. An object of the present invention is to provide a central processing device for a monitored-person monitoring system, a central processing method, and the monitored-person monitoring system which can assist in finding tendency in how to issue a notification (information).

The central processing device and the central processing method according to the present invention manage an event detected by a sensor device provided in correspondence with a monitored person and notify a predetermined terminal device of the event. The central processing device and the central processing method store the event received from the sensor device as monitoring information associated with the monitored person corresponding to the sensor device. The monitoring information includes a detection time or a reception time of the event and a type of the event. The central processing device and the central processing method extract a type of an event corresponding to a predetermined monitored person within a predetermined period from the stored monitoring information and make a predetermined display device display the events in the predetermined period by type and in association with the monitored person on the basis of the extraction result. The monitored-person monitoring system according to the present invention includes the central processing device.

The above and other objects, features, and advantages of the present invention will be apparent from the following detailed description and the attached drawings.

### Brief Description of Drawings

Fig. 1 is a diagram of a configuration of a monitored-person monitoring system according to an embodiment.
Fig. 2 is a diagram of a configuration of a sensor device in the monitored-person monitoring system.
Fig. 3 is a diagram of representative waveform patterns of normal respiration and abnormal respiration.
Fig. 4 is a diagram of a configuration of a management server device in the monitored-person monitoring system.
Fig. 5 is a diagram of a configuration of a monitoring information table stored in the management server device.
Fig. 6 is a diagram of a configuration of an inter-device information table stored in the management server device.
Fig. 7 is a diagram of a configuration of a sensor information table stored in the management server device.
Fig. 8 is a flowchart of an operation of the sensor device in the monitored-person monitoring system.
Fig. 9 is a flowchart of an operation of the management server device in the monitored-person monitoring system.
Fig. 10 is a flowchart of processing on monitoring information in the flowchart illustrated in Fig. 9.
Fig. 11 is a diagram of an example of a monitoring information screen displayed on a mobile terminal device in the monitored-person monitoring system.
Fig. 12 is a flowchart of processing for displaying personal history in the flowchart in Fig. 9.
Fig. 13 is a diagram of an example of a personal history display screen in a time chart displayed on a fixed terminal device in the monitored-person monitoring system.
Fig. 14 is a diagram of an example of a personal history display screen in a graph displayed on the fixed terminal device in the monitored-person monitoring system.
Fig. 15 is a flowchart of processing for displaying personal analysis in the flowchart in Fig. 9.
Fig. 16 is a diagram of an example of a personal analysis display screen displayed on the fixed terminal device in the monitored-person monitoring system.

### Description of Embodiments

Hereinafter, an embodiment of the present invention will be described with reference to the drawings. Note that components denoted with the same reference numerals in the drawings respectively indicate the same components, and description thereof will be appropriately omitted. Here, in a case of being collectively called, the components are indicated by a reference numeral of which a suffix is omitted, and in a case where an individual component is indicated, the component is indicated by a reference numeral with a suffix.

A monitored-person monitoring system according to the embodiment monitors a monitored person (target person to be watched) Ob who is a monitoring target (watching target) to be monitored (watched) and includes a sensor device which is provided in correspondence with the monitored person Ob and detects a predetermined event, which has been preset, related to the monitored person, a central processing device which is communicably connected to the sensor device and manages the event which has been detected by and received from the sensor device, and a terminal device which is communicably connected to the central processing device and receives a notification of the event detected by the sensor device via the central processing device. The central processing device includes a storage unit, a history information extraction processing part, and a monitoring processing part. The storage unit stores the event, which has been detected by and received from the sensor device, as monitoring information associated with the monitored person corresponding to the sensor device. The monitoring information includes a detection time or a reception time of the event and a type of the event. The history information extraction processing part extracts a type of an event corresponding to a predetermined monitored person within a predetermined period from the monitoring information stored in the storage unit. The monitoring processing part makes a predetermined display device display the events in the predetermined period by type and in association with the monitored person on the basis of the extraction result of the history information extraction processing part. The terminal device may be a single type of device. However, in the present embodiment, the terminal devices are two kinds of devices, i.e., a fixed terminal device and a mobile terminal device. A main difference between the fixed terminal device and the mobile terminal device is that the fixed terminal device is fixedly operated while the mobile terminal device is operated as being carried by a surveillant (user) such as a nurse and a caregiver. The fixed terminal device and the mobile terminal device are substantially the same.

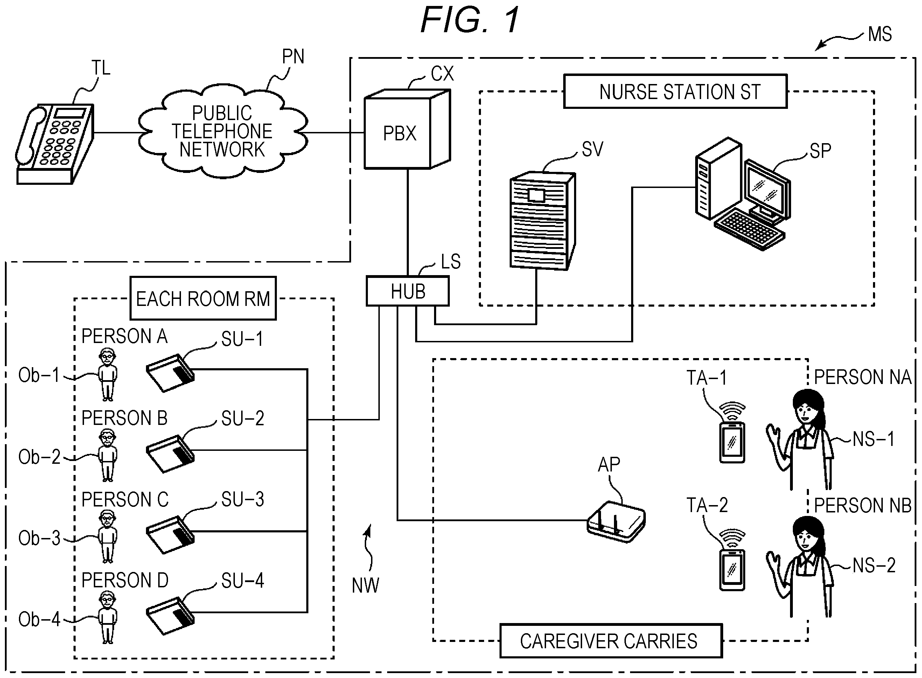

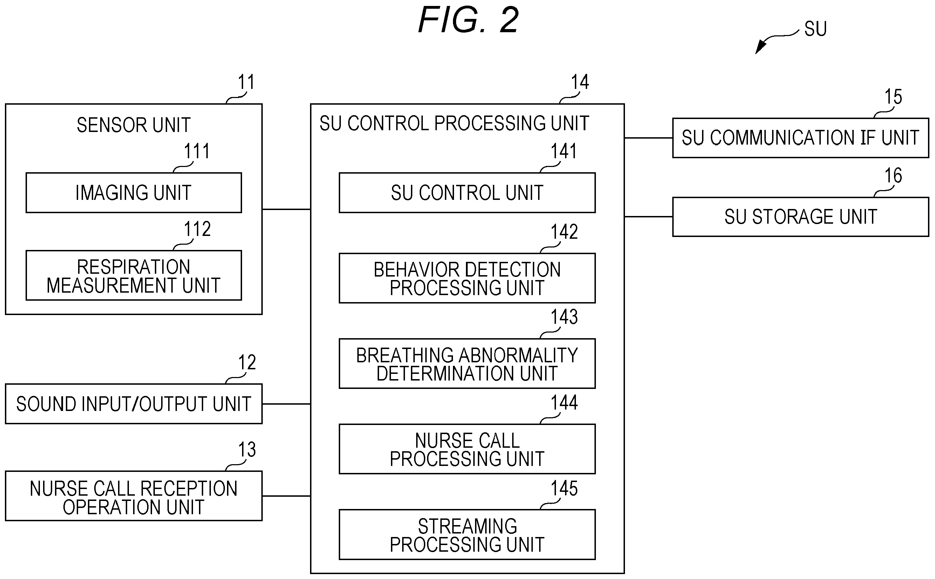

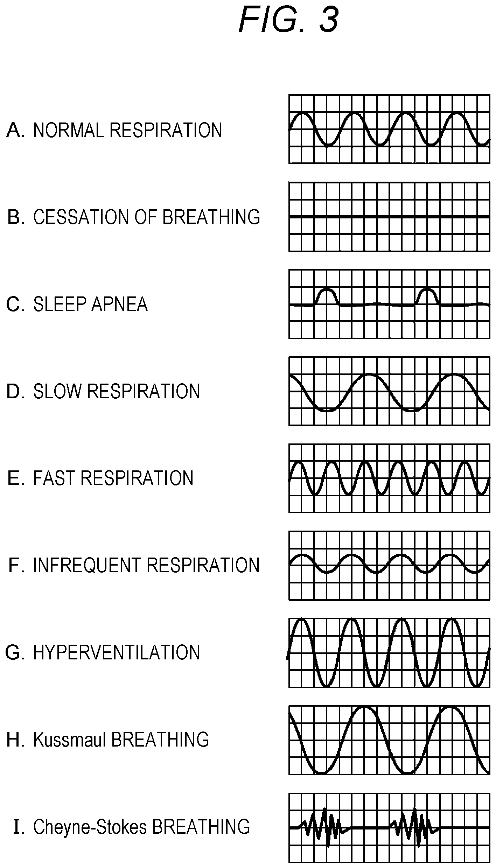

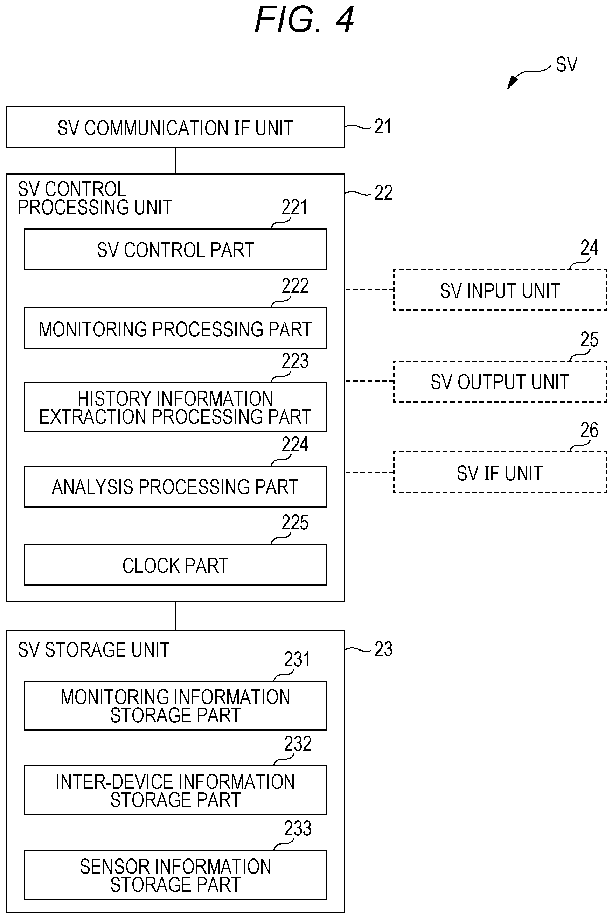

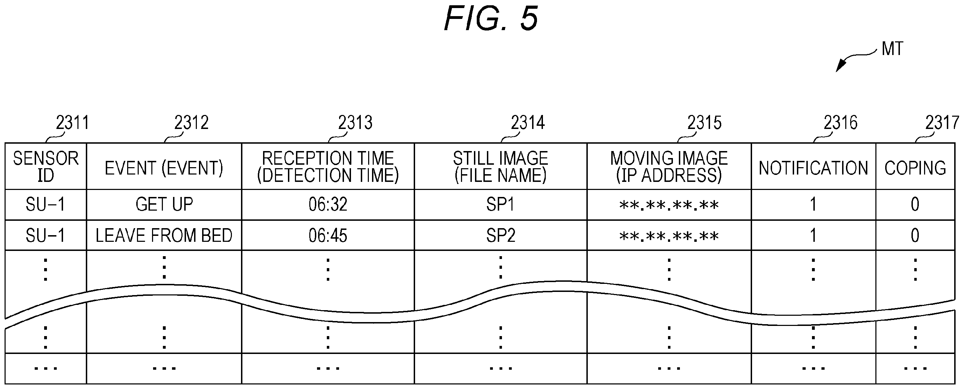

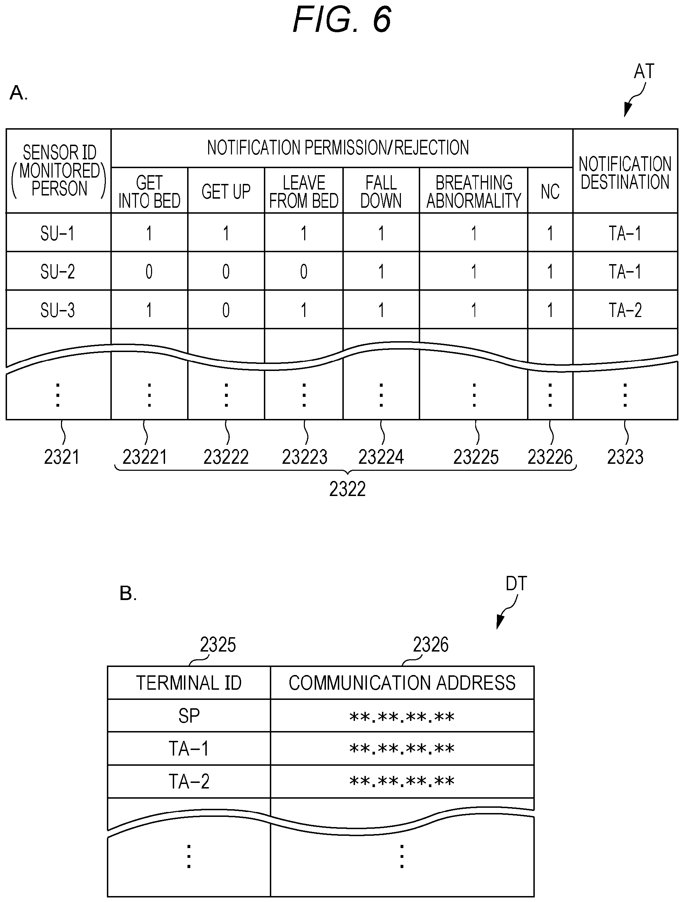

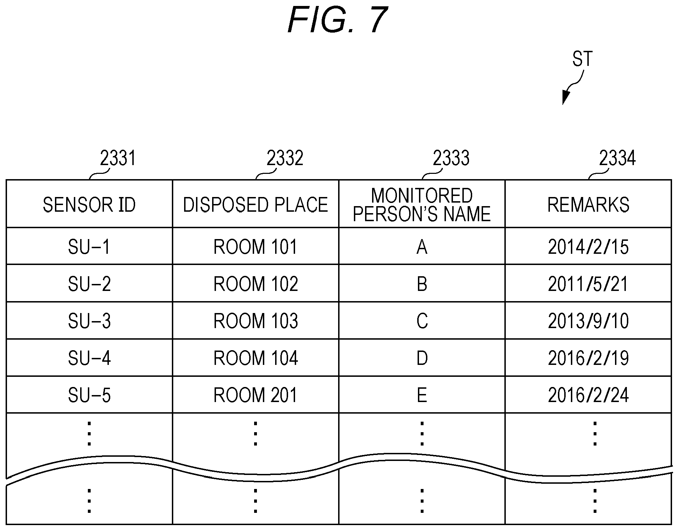

Fig. 1 is a diagram of a configuration of the monitored-person monitoring system according to the embodiment. Fig. 2 is a diagram of a configuration of the sensor device in the monitored-person monitoring system according to the embodiment. Fig. 3 is a diagram of representative waveform patterns of normal respiration and abnormal respiration. Fig. 3A indicates a representative waveform pattern of normal respiration, Fig. 3B indicates a representative waveform pattern of cessation of breathing, Fig. 3C indicates a representative waveform pattern of sleep apnea, Fig. 3D indicates a representative waveform pattern of slow respiration, Fig. 3E indicates a representative waveform pattern of fast respiration, Fig. 3F indicates a representative waveform pattern of infrequent respiration, Fig. 3G indicates a representative waveform pattern of hyperventilation, Fig. 3H indicates a representative waveform pattern of Kussmaul breathing, and Fig. 3I indicates a Cheyne-Stokes breathing. Fig. 4 is a diagram of a configuration of a management server device in the monitored-person monitoring system according to the embodiment. Fig. 5 is a diagram of a configuration of a monitoring information table stored in the management server device illustrated in Fig. 4. Fig. 6 is a diagram of a configuration of an inter-device information table stored in the management server device illustrated in Fig. 4. Fig. 6A is a configuration of a notification destination correspondence information table in the inter-device information table, and Fig. 6B is a configuration of a communication address correspondence information table in the inter-device information table. Fig. 7 is a diagram of a configuration of a sensor information table stored in the management server device illustrated in Fig. 4.

More specifically, for example, as illustrated in Fig. 1, a monitored-person monitoring system MS includes a single or a plurality of sensor devices SU (SU-1 to SU-4), a management server device SV, a fixed terminal device SP, a single or a plurality of mobile terminal devices TA (TA-1 and TA-2), and a private branch exchange (PBX) CX, and these devices are communicably connected to each other via a network (network and communication line) NW such as a local area network (LAN) in a wired or wireless manner. The network NW may include a relay such as a repeater, a bridge, and a router which relays communication signals. In the example illustrated in Fig. 1, the plurality of sensor devices SU-1 to SU-4, the management server device SV, the fixed terminal device SP, the plurality of mobile terminal devices TA-1 and TA-2, and the private branch exchange CX are communicably connected to each other with the LAN (for example, LAN supported by IEEE802.11 standard) NW which includes an L2 switch concentrator (hub, HUB) LS and an access point AP and in which wired communication and wireless communication are mixed. More specifically, the plurality of sensor devices SU-1 to SU-4, the management server device SV, the fixed terminal device SP, and the private branch exchange CX are connected to the concentrator LS, and the plurality of mobile terminal devices TA-1 and TA-2 are connected to the concentrator LS via the access point AP. Then, the network NW configures a so-called intranet by using a group of Internet protocols such as the Transmission control protocol (TCP) and the Internet protocol (IP).

The private branch exchange (line switch) CX is connected to the network NW, performs an extension call between the mobile terminal devices TA by controlling extension calls such as outgoing call, incoming call, and a call between the mobile terminal devices TA, is connected to an external telephone TL, for example, a fixed telephone and a mobile telephone via a public telephone network PN such as a fixed telephone network or a mobile telephone network, and performs an outside call between the external telephone TL and the mobile terminal device TA by controlling the outside call such as outgoing call, incoming call, and a call between the external telephone TL and the mobile terminal device TA. The private branch exchange CX is, for example, a digital exchange and an Internet Protocol Private Branch eXchange (IP-PBX).

The monitored-person monitoring system MS is disposed at an appropriate place according to the monitored person Ob. The monitored person (target person to be watched) Ob is, for example, a person who needs nursing due to sickness, injury, and the like, a person who needs nursing care due to a decline in physical ability and the like, and a person who lives alone. In particular, in terms of enabling early detection and early treatment, it is preferable that the monitored person Ob is a person who needs to be found when a predetermined unfavorable event such as an abnormal state occurs in the person. Therefore, the monitored-person monitoring system MS is preferably disposed in a building such as a hospital, a welfare facility for the aged, and a house according to the type of the monitored person Ob. In the example illustrated in Fig. 1, the monitored-person monitoring system MS is disposed in a building of a nursing facility including a plurality of rooms RM where a plurality of monitored persons Ob lives, a nurse station, and the like.

The sensor device SU is a device which has a communication function for communicating with the other devices SV, SP, and TA via the network NW, detects a predetermined event related to the monitored person Ob and notifies the management server device SV of the detected event, performs audio communication with the terminal devices SP and TA, and generates an image including a moving image to distribute the moving image to the terminal devices SP and TA. The sensor device SU is disposed in correspondence with each monitored person Ob. The predetermined event preferably includes an event which needs to be handled (coped with). In the present embodiment, the predetermined event includes a predetermined behavior which is preset of the monitored person Ob, and further includes breathing abnormality and a nurse call. For example, as illustrated in Fig. 2, such a sensor device SU includes a sensor unit 11, a sound input/output unit 12, a nurse call reception operation unit 13, a sensor control processing unit (SU control processing unit) 14, a sensor communication interface unit (SU communication IF unit) 15, and a sensor storage unit (SU storage unit) 16.

The sensor unit 11 is connected to the SU control processing unit 14 and detects a predetermined amount regarding the monitored person Ob which has been preset according to control of the SU control processing unit 14. The predetermined amounts are appropriately determined according to a monitoring item relative to the monitored person Ob. In the present embodiment, the monitoring item includes the predetermined behavior which has been preset and breathing abnormality, and the predetermined behavior includes four behaviors of the monitored person Ob, for example, to get into the bed, to get up, to leave the bed, and to fall down. To fall down may include a behavior that the monitored person Ob falls from the bedding, or the predetermined behavior may include to fall from the bedding in addition to falling down. In order to detect the predetermined behavior, the sensor unit 11 includes, for example, an imaging unit 111 and a respiration measurement unit 112 to detect the breathing abnormality.

The imaging unit 111 is a device which is connected to the SU control processing unit 14 and generates an image (image data) according to the control of the SU control processing unit 14. The image includes a still image (still image data) and a moving image (moving image data). The imaging unit 111 is arranged so as to monitor a space where the monitored person Ob is scheduled to be (location space, room RM which is a disposed place in the example illustrated in Fig. 1), images the location space from the above as an imaging target, generates a bird's-eye image of the imaging target (image data), and outputs the image of the imaging target (target image) to the SU control processing unit 14. Preferably, since probability that the entire monitored person Ob can be imaged is high, the imaging unit 111 is disposed so as to image the imaging target from just above a preset head planned position where the head of the monitored person Ob is planned to be positioned (normally, position where pillow is disposed) on the bedding where the monitored person Ob lies (for example, bed). The sensor device SU obtains the image of the monitored person Ob imaged from the above of the monitored person Ob by the imaging unit 111, preferably, an image imaged immediately above the head planned position. The sensor device SU outputs the generated image of the monitored person Ob (target image) to the SU control processing unit 14.

Such an imaging unit 111 may be a device for generating an image of visible light. However, in the present embodiment, the imaging unit 111 is a device for generating an image of infrared ray so as to monitor the monitored person Ob in a relatively dark state. In the present embodiment, for example, the imaging unit 111 is a digital infrared camera which includes an image forming optical system which forms an infrared optical image of the imaging target on a predetermined image forming surface, an image sensor which is arranged so that a light receiving surface matches the image forming surface and converts the infrared optical image of the imaging target into an electrical signal, an image processing unit which generates image data which is data indicating the infrared image of the imaging target by performing image processing on the output of the image sensor, and the like. In the present embodiment, the image forming optical system of the imaging unit 111 is preferably a wide-angle optical system (so-called wide-angle lens (including fisheye lens)) having an angle of view capable of imaging the entire room RM where the image forming optical system is disposed.

The respiration measurement unit 112 is a device which is connected to the SU control processing unit 14 and measures respiration of the monitored person Ob according to the control of the SU control processing unit 14. In the present embodiment, the measurement of the respiration is indirectly performed in a non-contact manner by measuring a movement of a body surface of the chest accompanying the respiration, and for example, the respiration measurement unit 112 includes a Doppler sensor. The Doppler sensor is a body motion sensor which transmits a transmission wave, receives a reflected wave of the transmission wave reflected by an object, and outputs a Doppler signal having a Doppler frequency component on the basis of the transmission wave and the reflected wave. In a case where the object is moving, a frequency of the reflected wave is shifted in proportion to a moving speed of the object by a so-called Doppler effect. Therefore, a difference between a frequency of the transmission wave and a frequency of the reflected wave (Doppler frequency component) occurs. The Doppler sensor generates a signal having the Doppler frequency component as a Doppler signal (measurement result of respiration measurement unit 112) and outputs the generated signal to the SU control processing unit 14. The transmission wave may be an ultrasonic wave, a microwave, and the like. In the present embodiment, the transmission wave is a microwave. Since the microwave can pass through clothing and can be reflected by the body surface of the monitored person Ob, the microwave is preferable because the movement of the body surface of the chest accompanying the respiration can be detected even when the monitored person Ob wears clothes.

The sound input/output unit 12 is a circuit which is connected to the SU control processing unit 14, obtains external sounds to input the sound to the sensor device SU, and generates and outputs sound according to the electric signal indicating sound under the control of the SU control processing unit 14. The sound input/output unit 12 includes, for example, a microphone which converts acoustic vibration of the sound into an electric signal and a speaker which converts the electric signal of the sound into the acoustic vibration of the sound. The sound input/output unit 12 outputs the electric signal indicating the external sound to the SU control processing unit 14 and converts the electric signal input from the SU control processing unit 14 into the acoustic vibration of the sound and outputs the converted signal.

The nurse call reception operation unit 13 is a switch circuit such as a push-button-type switch which is connected to the SU control processing unit 14 and inputs a nurse call to the sensor device SU. The nurse call reception operation unit 13 may be wiredly connected to the SU control processing unit 14 and may be connected to the SU control processing unit 14 by short-range wireless communication, for example, Bluetooth (registered trademark) standard.

The SU communication IF unit 15 is a communication circuit which is connected to the SU control processing unit 14 and performs communication according to the control of the SU control processing unit 14. The SU communication IF unit 15 generates a communication signal which stores data input from the SU control processing unit 14 and to be transferred according to a communication protocol used in the network NW of the monitored-person monitoring system MS and transmits the generated communication signal to the other devices SV, SP, and TA via the network NW. The SU communication IF unit 15 receives the communication signal from the other devices SV, SP, and TA via the network NW, extracts data from the received communication signal, and converts the extracted data into data in a format which can be processed by the SU control processing unit 14, and outputs the converted data to the SU control processing unit 14. The SU communication IF unit 15 includes, for example, a communication interface circuit supported by the IEEE802.11 standard.

The SU storage unit 16 is a circuit which is connected to the SU control processing unit 14 and stores predetermined various programs and predetermined various data according to the control of the SU control processing unit 14. The various predetermined programs include, for example, an SU control program for controlling each part of the sensor device SU according to the function of each part, a behavior detection processing program for detecting a predetermined behavior of the monitored person Ob on the basis of the target image of the imaging unit 111 in the sensor unit 11 and notifying the management server device SV of the detected behavior, a breathing abnormality determination program for determining whether the breathing of the monitored person is abnormal on the basis of the measurement result of the respiration measurement unit 112 in the sensor unit 11 and notifying the management server device SV of the breathing abnormality, a nurse call processing program for notifying the management server device SV that the nurse call has been received by the nurse call reception operation unit 13 in a case where the nurse call has been received by the nurse call reception operation unit 13 and performing audio communication with the terminal devices SP and TA by using the sound input/output unit 12 and the like, and a control processing program such as a streaming processing program for distributing the moving image generated by the imaging unit 111 to the terminal devices SP and TA which have requested the moving image by streaming. The predetermined various data includes a sensor device identifier (sensor ID) of the system which is an identifier to specify and identify the sensor device SU, data necessary for executing each program such as a communication address of the management server device SV, and the like. The SU storage unit 16 includes, for example, a read only memory (ROM) which is a nonvolatile storage element and an electrically erasable programmable read only memory (EEPROM) which is rewritable nonvolatile storage element. The SU storage unit 16 includes a random access memory (RAM) to be a so-called working memory of the SU control processing unit 14 which stores data generated during the execution of the predetermined program, and the like.

The SU control processing unit 14 is a circuit which controls each unit of the sensor device SU according to the function of each unit, detects a predetermined event related to the monitored person Ob and notifies the management server device SV of the detected event, performs audio communication with the terminal devices SP and TA, and generates an image including a moving image to distribute the moving image to the terminal devices SP and TA. The SU control processing unit 14 includes, for example, a central processing unit (CPU) and peripheral circuits. By executing the control processing program, the SU control processing unit 14 functionally includes a sensor control unit (SU control unit) 141, a behavior detection processing unit 142, a breathing abnormality determination unit 143, a nurse call processing unit 144, and a streaming processing unit 145.

The SU control unit 141 controls each unit of the sensor device SU according to the function of each unit and integrally controls the sensor device SU.

The behavior detection processing unit 142 detects a predetermined behavior of the monitored person Ob which has been preset on the basis of the target image generated by the imaging unit 111 of the sensor unit 11 and notifies the management server device SV of the detected behavior. More specifically, in the present embodiment, the predetermined behavior includes four behaviors, i.e., to get into the bed, to get up, to leave the bed, and to fall down. Then, for example, the behavior detection processing unit 142 detects a head of the monitored person Ob on the basis of the target image imaged by the imaging unit 111 to detect that the monitored person Ob gets up and falls down on the basis of a temporal change in the size of the detected head of the monitored person Ob and detects the monitored person Ob on the basis of the target image imaged by the imaging unit 111 to detect that the monitored person Ob leaves the bed and gets into the bed on the basis of the overlapped state of the detected monitored person Ob and the bedding. More specifically, first, a location region of bedding BD, a first threshold Th1 and a second threshold Th2 are stored in advance in the SU storage unit 16 as one piece of the predetermined various data. The first threshold Th1 is a value for discriminating the size of the head of the monitored person Ob in a lying posture and the size of the head of the monitored person Ob in a sitting posture in the location region of the bedding BD. The first threshold Th1 is a value for discriminating whether the size of the head is the size of the head of the monitored person Ob in the lying posture in the room RM except for the location region of the bedding BD. The behavior detection processing unit 142 extracts a moving body region as a region of a person who is the monitored person Ob from the target image, for example, by a background difference method and a frame difference method. Next, the behavior detection processing unit 142 extracts the head region of the monitored person Ob from the extracted moving body region by performing Hough transformation on a circle and an oval, performing pattern matching using a model of the head which has been previously prepared, or by a neural network which has learned for detection of the head. Then, the behavior detection processing unit 142 detects getting up and to falling down on the basis of the position and the size of the extracted head. For example, in a case where the position of the extracted head is within the location region of the bedding BD and the size of the extracted head has been temporally changed from the size at the time of the lying posture to the size at the time of the sitting posture by using the first threshold Th1, the behavior detection processing unit 142 determines and detects that the monitored person Ob has got up. For example, in a case where the position of the extracted head is within the room RM except for the location region of the bedding BD and the size of the extracted head has been temporally changed from a certain size to the size at the time of the lying posture by using the second threshold Th2, the behavior detection processing unit 142 determines and detects that the monitored person Ob has fallen down. Then, the behavior detection processing unit 142 detects an overlapping region where the extracted moving body region and the location region of the bedding BD from the target image as described above and detects that the monitored person Ob has left the bed and has got into the bed on the basis of the temporal change in the overlapping region. For example, in a case where the state has temporally changed from a state where the detected overlapping region exists to a state where the overlapping region does not exist, the behavior detection processing unit 142 determines and detects that the monitored person Ob has left the bed. For example, in a case where a state has been temporally changed from a state where the detected overlapping region does not exist to a state where the entire overlapping region is within the location region of the bedding BD after the detection of the overlapping region (that is, state where moving body region is completely contained in location region of bedding BD), the behavior detection processing unit 142 determines and detects that the monitored person Ob has got into the bed.

When detecting the predetermined behavior in this way, the behavior detection processing unit 142 makes the SU communication IF unit 15 notify the management server device SV of a first event notification communication signal to issue a notification of the event which stores event information (event information) indicating a content of a predetermined event related to the monitored person Ob. More specifically, the behavior detection processing unit 142 transmits a communication signal (first event notification communication signal) which stores a sensor ID of the system, the event information indicating the content of the event, and a target image related to the event to the management server device SV via the SU communication IF unit 15. In the present embodiment, the event information indicates one or a plurality of behaviors, i.e., to get into the bed, to get up, to leave the bed, to fall down, the breathing abnormality, and the nurse call (NC), and here, the behavior detection processing unit 142 stores the one or the plurality of events from among to get into the bed, to get up, to leave the bed, and to fall down which has been detected in the first event notification communication signal as the event information. Here, for example, the target image related to the event is an image used to detect the predetermined behavior. The target image may be at least one of a still image and a moving image. In the present embodiment, a still image is given in notification first, and a moving image is distributed in response to a request of a user. Note that a moving image may be distributed first, or it is possible that a still image and a moving image are transmitted and the still image and the moving image are displayed on the terminal devices SP and TA by dividing a screen.

The breathing abnormality determination unit 143 determines whether respiration of the monitored person is abnormal on the basis of the measurement result of the respiration measurement unit 112 of the sensor unit 11 and notifies the management server device SV of the breathing abnormality. The breathing abnormality is respiration indicating an abnormal waveform pattern with respect to the normal respiration of the waveform pattern illustrated in Fig. 3A, for example, in the present embodiment, the breathing abnormality includes cessation of breathing (Fig. 3B), sleep apnea (Fig. 3C), slow respiration (Fig. 3D), fast respiration (Fig. 3E), infrequent respiration (Fig. 3F), hyperventilation (Fig. 3G), Kussmaul breathing (Fig. 3H), and Cheyne-Stokes breathing (Fig. 3I). The cessation of breathing indicates a case where respiration is stopped for 30 seconds. The sleep apnea indicates a case where cessation of breathing for equal to or longer than 10 seconds is observed equal to or more than five times per hour during sleep. The slow respiration indicates a case where the number of respirations per minute is equal to or less than 12 times. The fast respiration indicates a case where the number of respirations per minute is equal to or more than 24 times. The infrequent respiration indicates a case where an amplitude of the body motion is smaller than a normal case in a state of shallow breathing (for example, amplitude equal to or less than 40% of average value in past one month is detected). The hyperventilation indicates a case where the amplitude of the body motion is larger than a normal case in a state of deep breathing (for example, increase of 40% than average value in past one month is detected). The Kussmaul breathing indicates a case where the number of respirations per minute is equal to or less than 12 times and the amplitude of the body motion is larger than a normal case (for example, equal to or larger than 40% larger amplitude than average value in past one month is detected). The Cheyne-Stokes breathing indicates a case where the amplitude of the body motion is increased each time (for example, 1.5 times at maximum), and then, decreased, and after that, a state where an apnea state continues for a predetermined time (for example, 20 seconds to one minute) is repeated. The cessation of breathing, the sleep apnea, the slow respiration, the fast respiration, the infrequent respiration, the hyperventilation, the Kussmaul breathing, and the Cheyne-Stokes breathing are detected by a known conventional method on the basis of the measurement result of the respiration measurement unit 112 (Doppler signal of Doppler sensor in present embodiment).

When detecting the breathing abnormality in this way, the breathing abnormality determination unit 143 notifies the management server device SV of the first event notification communication signal which stores the detection of the breathing abnormality as another example of the predetermined event. More specifically, the breathing abnormality determination unit 143 transmits the first event notification communication signal which stores the sensor ID of the system, the breathing abnormality as the event information, and the target image related to the event to the management server device SV via the SU communication IF unit 15. Here, the target image related to the event is, for example, an image which is generated by the imaging unit 111 of the sensor unit 11 when the breathing abnormality is detected.

When the nurse call reception operation unit 13 has received a nurse call, the nurse call processing unit 144 notifies the management server device SV of the first event notification communication signal which stores the reception of the nurse call as another example of the predetermined event and performs audio communication with the terminal devices SP and TA by using the sound input/output unit 12 and the like. More specifically, when an input operation is performed on the nurse call reception operation unit 13, the nurse call processing unit 144 transmits the first event notification communication signal which stores the sensor ID of the system and the nurse call as the event information to the management server device SV via the SU communication IF unit 15. The first event notification communication signal which stores the nurse call as the event information may further store an image generated by the imaging unit 111 of the sensor unit 11 when the nurse call reception operation unit 13 has received the nurse call as the target image related to the event. Then, the nurse call processing unit 144, for example, performs audio communication with the terminal devices SP and TA by the Voice over Internet Protocol (VoIP) by using the sound input/output unit 12 and the like.

In a case where a request for distribution of the moving image is issued from the fixed terminal device SP or the mobile terminal devices TA via a communication IF unit 3, the streaming processing unit 145 distributes the moving image (for example, live moving image) generated by the imaging unit 111 to the fixed terminal device SP or the mobile terminal devices TA which has requested the distribution via the SU communication IF unit 15 in streaming reproduction.

In Fig. 1, as an example, the first to fourth sensor devices SU-1 to SU-4 which are four devices are illustrated. The first sensor device SU-1 is disposed in a room RM-1 (not shown) of a person A Ob-1 who is one of the monitored persons Ob, the second sensor device SU-2 is disposed in a room RM-2 (not shown) of a person B Ob-2 who is one of the monitored persons Ob, the third sensor device SU-3 is disposed in a room RM-3 (not shown) of a person C Ob-3 who is one of the monitored persons Ob, and the fourth sensor device SU-4 is disposed in a room RM-4 (not shown) of a person D Ob-4 who is one of the monitored persons Ob.

The management server device SV has a communication function for communicating with the other devices SU, TA, and SP via the network NW. When receiving the notification of the predetermined event from the sensor device SU, the management server device SV manages information on monitoring of the monitored person Ob (monitoring information (for example, predetermined event in the present embodiment (predetermined behavior and breathing abnormality detected by sensor device SU, nurse call received by sensor device SU), image of monitored person Ob (still image and moving image), and time of reception of notification), notifies (re-notifies, re-informs, and transmits) the predetermined terminal devices SP and TA of the predetermined event, provides data in response to the request of the client (terminal devices SP and TA and the like in the present embodiment) to the client, and manages the entire monitored-person monitoring system MS. Then, in the present embodiment, the management server device SV extracts the type of the event corresponding to the predetermined monitored person Ob within a predetermined period from the monitoring information and displays the events in the predetermined period, for example, on the fixed terminal device SP by type and in association with the monitored person Ob, on the basis of the extraction result. For example, as illustrated in Fig. 4, such a management server device SV includes a server communication interface unit (SV communication IF unit) 21, a server control processing unit (SV control processing unit) 22, and a server storage unit (SV storage unit) 23.

As the SU communication IF unit 15, the SV communication IF unit 21 is a communication circuit which is connected to the SV control processing unit 22 and performs communication according to the control of the SV control processing unit 22. The SV communication IF unit 21 includes, for example, a communication interface circuit supported by the IEEE802.11 standard.

The SV storage unit 23 is a circuit which is connected to the SV control processing unit 22 and stores predetermined various programs and predetermined various data according to the control of the SV control processing unit 22. The predetermined various programs include, for example, a SV control program which controls each unit of the management server device SV according to the function of each unit, a SV monitoring processing program which executes predetermined information processing regarding monitoring of the monitored person Ob, a history information extraction processing program which extracts the type of the event corresponding to the predetermined monitored person Ob within a predetermined period from the monitoring information stored in the SV storage unit 23, an analysis processing program which executes predetermined analysis processing on the monitoring information stored in the SV storage unit 23, and a control processing program such as a clock program for measuring time. The predetermined various data includes data necessary for execution of each program such as a server identifier (server ID) which is an identifier for specifying and identifying the management server device SV, the monitoring information of the monitored person Ob, inter-device information indicating information between the devices SU, SP, and TA such as the notification destinations of the predetermined event, and sensor information regarding the sensor device SU. In order to store the monitoring information, the inter-device information, and the sensor information, the SV storage unit 23 functionally includes a monitoring information storage part 231, an inter-device information storage part 232, and a sensor information storage part 233.

The monitoring information storage part 231 stores the monitoring information of the monitored person Ob transmitted/received to/from the devices SU, SP, and TA. More specifically, in the present embodiment, as the monitoring information, the monitoring information storage part 231 stores the sensor ID, the event information (to get into bed, to get up, to leave bed, to fall down, breathing abnormality, and nurse call), the reception time, the target image (still image and moving image), whether the notification is issued and the countermeasures associated with each other, on the basis of the information stored in the communication signal such as the first event notification communication signal.

In the present embodiment, the monitoring information is stored in the monitoring information storage part 321 in a table format. For example, as illustrated in Fig. 5, a monitoring information table MT in which the monitoring information is registered includes a sensor ID field 2311 in which the sensor ID stored in the communication signal received from each of the devices SU, SP, and TA is registered, an event field (event field) 2312 in which the event information stored in the received communication signal is registered, a reception time field 2313 in which the reception time of the received communication signal is registered, a still image field 2314 in which the still image stored in the received communication signal is registered, a moving image field 2315 in which a communication address (for example, IP address) of the sensor device SU corresponding to the sensor ID stored in the received communication signal is registered as an acquisition destination of a live moving image, a notification field 2316 in which whether the notification regarding the event information stored in the received communication signal has been issued to the predetermined terminal devices SP and TAis registered, and a coping field 2317 in which the presence/absence of coping to the event information stored in the received communication signal is registered, and the monitoring information table MT includes a record for each of the received communication signals (for each event). In the still image field 2314, for example, the image data of the still image may be registered, and in addition, for example, a file name of the image data of the still image may be registered. In the notification field 2316, as will be described later, a flag (notification flag) indicating whether the event information stored in the received communication signal is given in notification (given in re-notification) to the predetermined terminal devices SP and TA is registered. For example, in the present embodiment, in the notification field 2316, a notification flag "1" which means that the event information stored in the received communication signal has been given in notification to the predetermined terminal devices SP and TA (notified) or a notification flag "0" which means that the event information stored in the received communication signal has not been given in notification to the predetermined terminal devices SP and TA (not notified) are registered. In the notification field 2316, the notification flag "0" which means "not notified" is registered as a default. In the coping field 2317, as will be described later, a flag (coping flag) is registered which indicates whether the terminal devices SP and TA have received that a surveillant (user) who uses the terminal device has an intention to make predetermined coping (cope with, treat, measure), for example, life saving, nursing, nursing care, and assistance relative to the event information stored in the received communication signal. For example, in the present embodiment, in the coping field 2317, the coping flag "1" which means that the terminal devices SP and TA have received (received) the intention to cope with the type of the predetermined behavior stored in the received communication signal as the event information, the breathing abnormality, and the nurse call (event information registered in event field 2312) or the coping flag "0" which means that the terminal devices SP and TA have not received (not received) the intention to cope with the event information stored in the received communication signal is registered. The coping flag "0" which means "not received" is registered in the coping field 2317 as a default.

In a case where the first event notification communication signal stores a detection time when the predetermined behavior is detected, a detection time when the breathing abnormality is detected, or a detection time when the nurse call is received and detected, the detection time may be registered instead of the reception time.

In the present embodiment, the inter-device information storage part 232 stores notification destination relationship, communication address correspondence relationship, and the like as the inter-device information. The notification destination relationship is correspondence relationship of the first event notification communication signal transmitted from the sensor device SU relative to a sensor ID which is a notification source (transmission source), permission/rejection of notification by type of the event, and a terminal ID which is a notification destination (transmission destination). The communication address correspondence relationship is correspondence relationship between the ID (sensor ID, terminal identifier (terminal ID)) of each of the devices SU, SP, and TA and the communication address. The terminal ID is an identifier for specifying and identifying the terminal devices SP and TA.

The notification destination relationship and the communication address correspondence relationship are stored in the inter-device information storage part 232 in a table format in the present embodiment. For example, as illustrated in Fig. 6A, a notification destination correspondence information table AT in which the notification destination relationship is registered includes a sensor ID field 2321 in which the sensor ID of the sensor device SU of the notification source (transmission source) is registered, a notification destination field 2323 in which the terminal IDs of the terminal devices SP and TA to be the notification destination (transmission destination) for receiving the notification of the event notified from the sensor device SU corresponding to the sensor ID registered in the sensor ID field 2321 are registered, and a notification permission/rejection field 2322 in which permission/rejection of the notification indicating whether a notification of the event given in notification from the sensor device SU corresponding to the sensor ID registered in the sensor ID field 2321 is issued (transmitted) to the terminal devices SP and TA corresponding to the terminal IDs registered in the notification destination field 2323 is registered. The notification destination correspondence information table AT includes a record for each sensor ID (sensor device SU). In the present embodiment, the permission/rejection of the notification can be set by type of the event. Therefore, in response to this, the notification permission/rejection field 2322 includes a plurality of subfields in which the permission/rejection of the notification is registered by type of the event. More specifically, the notification permission/rejection field 2322 includes a getting-into-bed subfield 23221 in which permission/rejection of the notification (transmission) of the event is registered in a case where the type of the event given in notification from the sensor device SU corresponding to the sensor ID registered in the sensor ID field 2321 is to get into the bed, a getting-up subfield 23222 in which permission/rejection of the notification of the event is registered in a case where the type of the event given in notification is to get up, a leaving-bed subfield 23223 in which permission/rejection of the notification of the event is registered in a case where the type of the event given in notification is to leave the bed, a falling-down subfield 23224 in which permission/rejection of the notification of the event is registered in a case where the type of the event given in notification is to fall down, a breathing abnormality subfield 23225 in which permission/rejection of the notification of the event is registered in a case where the type of the event given in notification is the breathing abnormality, and a nurse call subfield (NC subfield) 23226 in which permission/rejection of the notification of the event is registered in a case where the type of the event given in notification is the nurse call. In each of the notification permission/rejection fields 2322 (23221 to 23226), a flag indicating whether the event is given in notification (notification permission/rejection flag) is registered. For example, in the present embodiment, in the notification permission/rejection fields 2322 (23221 to 23226), a notification permission/rejection flag "1" which means that the notification of the event is permitted (notify) or a notification permission/rejection flag "0" which means that the notification of the event is not permitted (not notify) is registered. In each of the notification permission/rejection fields 2322 (23221 to 23226), the notification permission/rejection flag "1" which means the permission of the notification is registered as a default. Note that the notification destination correspondence information table AT corresponds to an example of notification permission/rejection information indicating whether the event detected by and received from the sensor device SU is given in notification to the predetermined terminal devices SP and TA by type of the event.

For example, as illustrated in Fig. 6B, the communication address correspondence information table DT in which the communication address correspondence relationship is registered includes an ID field 2325 in which IDs of the devices SU, SP, and TA are registered, a communication address field 2326 in which a communication address of each of the devices SU, SP, and TA corresponding to the ID registered in the ID field 2325 is registered, and includes a record for each ID (each of devices SU, SP, and TA).

The sensor ID, the server ID, and the terminal ID may be, for example, a serial number including a predetermined reference symbol string and the like, and may be a communication address (in this case, communication address correspondence relationship can be omitted).

The sensor information storage part 233 stores the sensor information. In the present embodiment, the sensor information is information on the sensor device SU and information in which the sensor ID of the sensor device SU is associated with a monitored person's name of the monitored person Ob.

In the present embodiment, the sensor information is stored in the sensor information storage part 233 in a table format. More specifically, for example, as illustrated in Fig. 7, a sensor information table ST in which the sensor information is registered includes a sensor ID field 2331 in which the sensor ID is registered, a disposed place field 2332 in which a disposed place of the sensor device SU having the sensor ID registered in the sensor ID field 2331 is registered, a monitored person's name field 2333 in which the monitored person's name of the monitored person Ob (that is, monitored person Ob who stays at the disposed place of the sensor device SU having the sensor ID registered in the sensor ID field 2331) monitored by the sensor device SU having the sensor ID registered in the sensor ID field 2331 is registered, and a remarks field 2334 in which remarks regarding the sensor device SU having the sensor ID registered in the sensor ID field 2331, the disposed place of the sensor device SU, and the monitored person Ob are registered, and includes a record for each sensor ID (that is, sensor device SU, in other words, monitored person Ob).

As described above, in the present embodiment, the monitoring information is stored in the SV storage unit 23 with relational using the sensor ID, and in the monitoring information, the event detected by and received from the sensor device SU is associated with the monitored person Ob (monitored person's name) corresponding to the sensor device SU.

The SV control processing unit 22 is a circuit which controls each unit of the management server device SV according to the function of each unit, manages the monitoring information regarding monitoring of the monitored person Ob when receiving the notification of the predetermined event from the sensor device SU, notifies (re-notifies, re-informs, and transmits) the predetermined terminal devices SP and TA of the predetermined event, provides data in response to a request of a client to the client, and manages the entire monitored-person monitoring system MS. In the present embodiment, the SV control processing unit 22 extracts the type of the event corresponding to the predetermined monitored person Ob within a predetermined period from the monitoring information and displays the events in the predetermined period, for example, on the fixed terminal device SP by type and in association with the monitored person Ob, on the basis of the extraction result. The SV control processing unit 22 includes, for example, a CPU and peripheral circuits. By executing the control processing program, the SV control processing unit 22 functionally includes a server control part (SV control part) 221, a monitoring processing part 222, a history information extraction processing part 223, an analysis processing part 224, and a clock part 225.

The SV control part 221 controls each unit of the management server device SV according to the function of each unit and integrally controls the management server device SV.

The monitoring processing part 222 manages the monitoring information regarding monitoring of the monitored person Ob when receiving the notification of the predetermined event from the sensor device SU and notifies the predetermined terminal devices SP and TA of the predetermined event on the basis of whether the notification corresponding to the type of the received event is permitted. More specifically, when receiving the first event notification communication signal from the sensor device SU, the monitoring processing part 222 stores (records) the monitoring information regarding monitoring of the monitored person Ob stored in the first event notification communication signal to the monitoring information storage part 231. Then, the monitoring processing part 222 searches the permission/rejection of the notification corresponding to the type of the event which corresponds to the sensor device SU which has transmitted the received first event notification communication signal and is indicated by the event information stored in the received first event notification communication signal from the notification destination relationship stored in the inter-device information storage part 232, does not transmit a second event notification communication signal and stores (records) that the second event notification communication signal has not been transmitted (given in notification) to the monitoring information storage part 231 in a case where the notification is not permitted as a result of the search. On the other hand, in a case where the notification is permitted as a result of the search, the monitoring processing part 222 selects (searches) a notification destination (re-notification destination, transfer destination, and transmission destination) corresponding to the sensor device SU which has transmitted the received first event notification communication signal from the notification destination relationship stored in the inter-device information storage part 232, transmits a second event notification communication signal to the selected terminal devices SP and TA, and stores (records) that the second event notification communication signal has been transmitted (given in notification) to the monitoring information storage part 231. The selection (search processing) is performed on the basis of the sensor ID corresponding to the sensor device SU which has transmitted the received first event notification communication signal. In a case where the event information stored in the first event notification communication signal is any one of the predetermined behavior (one of or some of to get into bed, to get up, to leave bed, and to fall down) and breathing abnormality, the second event notification communication signal stores the sensor ID, the event information, and the target image stored in the first event notification communication signal and a communication address corresponding to the sensor device SU having the sensor ID stored in the first event notification communication signal as a moving image download destination. The communication address is selected (searched) from the communication address correspondence relationship on the basis of the sensor ID corresponding to the sensor device SU which has transmitted the received first event notification communication signal. In a case where the event information stored in the first event notification communication signal is breathing abnormality, the sensor ID, the event information (breathing abnormality), and the target image stored in the first event notification communication signal are stored in the second event notification communication signal. In a case where the event information stored in the first event notification communication signal is the nurse call, the sensor ID and the event information (nurse call) stored in the first event notification communication signal are stored in the second event notification communication signal.

The history information extraction processing part 223 extracts the type of the event corresponding to the predetermined monitored person Ob in the predetermined period from the monitoring information stored in the monitoring information storage part 231 of the SV storage unit 23. Then, the monitoring processing part 222 further makes a predetermined display device display the events in the predetermined period by type and in association with the monitored person Ob on the basis of the extraction result of the history information extraction processing part 223. The monitoring processing part 222 makes the display device further display whether the event is given in notification when making the predetermined display device display the event.

The predetermined period and the predetermined monitored person Ob used by the history information extraction processing part 223 are input from outside and designated. For example, the predetermined display device further includes an input unit, and the predetermined period and the predetermined monitored person Ob are input from the input unit and designated. For example, in a case where the management server device SV includes a server input unit (SV input unit) 24 and a server output unit (SV output unit) 25 to be described later as indicated by a broken line in Fig. 4, the predetermined display device may be the SV output unit 25, and the predetermined period and the predetermined monitored person Ob are input from the SV input unit 24 and designated. In the present embodiment, for example, the predetermined display device is a display device communicably connected to the management server device SV such as the terminal devices SP and TA, and here, the predetermined display device is the fixed terminal device SP. The predetermined period and the predetermined monitored person Ob are input from the fixed terminal device SP by a surveillant (user), and the fixed terminal device SP transmits a monitored person's name of the predetermined monitored person Ob which has been input and designated (or sensor ID of sensor device SU for monitoring the predetermined monitored person Ob) and a communication signal which stores the input and designated predetermined period (for example, personal history request communication signal and personal analysis request communication signal to be described later) to the management server device SV. The history information extraction processing part 223 extracts the type of the event corresponding to the predetermined monitored person Ob in the predetermined period stored in the personal history request communication signal from the monitoring information stored in the monitoring information storage part 231 of the SV storage unit 23. Then, the monitoring processing part 222 transmits the communication signal (for example, history information notification communication signal and analysis information notification communication signal to be described later) which stores the type of the event and the monitored person's name of the monitored person Ob extracted by the history information extraction processing part 223 in the predetermined period to the predetermined display device (here, fixed terminal device SP) and makes the predetermined display device (here, fixed terminal device SP) display the events in the predetermined period by type and in association with the predetermined monitored person Ob. The predetermined period is appropriately designated, for example, one day, one week, one month, and three months.

The analysis processing part 224 executes predetermined analysis processing on the monitoring information stored in the monitoring information storage part 231 of the SV storage unit 23. More specifically, for example, the analysis processing part 224 obtains a time from getting into the bed to leaving the bed as a lying time on the basis of the extraction result of the history information extraction processing part 223 and executes first analysis processing in which a time from first time to get into the bed on or after a predetermined first time to a fist time to leave the bed on or after a predetermined second time on the next day from the obtained lying time is assumed as a sleeping time. Each of the predetermined first time and the predetermined second time is a time to retrieve a sleeping time at night and is appropriately set. The predetermined first time is set to, for example, 20 o'clock, 21 o'clock, or 22 o'clock, and the predetermined second time is set to, for example, five o'clock, six o'clock, or seven o'clock. In the present embodiment, for example, the predetermined first time is set to 20 o'clock, and the predetermined second time is set to five o'clock.

When the monitoring processing part 222 makes the predetermined display device (here, fixed terminal device SP) display the events in the predetermined period by type and in association with the monitored person Ob on the basis of the extraction result of the history information extraction processing part 223, the monitoring processing part 222 makes the predetermined display device (here, fixed terminal device SP) further display a sleeping time (night sleeping time) obtained by the analysis processing part 224 and the lying time excluding the sleeping time at night (daytime sleeping time) in different display modes. For example, the sleeping time and the lying time excluding the sleeping time are displayed on the predetermined display device with different display colors. In one example, the sleeping time is displayed in blue, and the lying time excluding the sleeping time is displayed in yellow. In addition, for example, the sleeping time and the lying time excluding the sleeping time are displayed on the predetermined display device with different lighting states. In one example, the sleeping time is displayed by continuous lighting, and the lying time excluding the sleeping time is displayed by blinking.

The analysis processing part 224 further executes second analysis processing, in which the analysis processing part 224 obtains the first counting result by counting the events corresponding to the predetermined monitored person Ob by type within the predetermined second period which is an analysis target on the basis of the monitoring information stored in the monitoring information storage part 231, obtains the second counting result by counting the events corresponding to the predetermined monitored person Ob by type in a predetermined third period before the predetermined second period and obtaining an average value according to the second period length, compares the first counting result and the second counting result to obtain the comparison result by type which changes by equal to or more than a predetermined threshold. In the present embodiment, for example, the predetermined second period includes a plurality of sub-periods, and the analysis processing part 224 executes the second analysis processing in which each of the plurality of sub-periods for each monitored person Ob is assumed as the second period. Furthermore, for example, the analysis processing part 224 executes the second analysis processing on each of the plurality of monitored persons Ob. The monitoring processing part 222 makes the display device display the comparison result by type that changes by equal to or more than the predetermined threshold obtained by executing the second analysis processing. For example, the monitoring processing part 222 makes the predetermined display device display the comparison result by type which changes by equal to or more than the predetermined threshold obtained by executing the second analysis processing and the comparison result by type which does not change by equal to or more than the predetermined threshold obtained by executing the second analysis processing in display modes different from each other. For example, as described above, the comparison results are displayed with different colors, different lighting states, and the like.

The predetermined second period and the predetermined monitored person Ob used in the second analysis processing are input from outside and are designated. For example, as described above, for example, the predetermined second period and the predetermined monitored person Ob may be input from the SV input unit 24. In addition, the predetermined second period and the predetermined monitored person Ob may be input, for example, from the fixed terminal device SP and the mobile terminal device TA. The predetermined second period is appropriately designated, for example, as one day, one week, one month, and three months. The sub-period is appropriately designated according to the predetermined second period, for example, one day, one week, one month, and three months ((length of second period) ≥ (length of sub-period)). For example, in a case where the second period is one week, the sub-period is one day. Furthermore, for example, in a case where the second period is one month, the sub-period is one week or one day. In the present embodiment, the sub-period is set to one day as a default. The predetermined third period is appropriately set in advance, for example, to one day, one week, one month, and three months. Preferably, the predetermined third period is changed according to the predetermined second period. In addition, preferably, the predetermined second period and the predetermined third period are the same period lengths. For example, in a case where the predetermined second period is one day, the predetermined third period is one week or one day. Furthermore, for example, in a case where the predetermined second period is one week, the predetermined third period is one month or one week. In the present embodiment, for example, the predetermined third period is set to one week as a default. The predetermined third period may be input from outside and designated. The predetermined threshold is appropriately set by type of the event in advance, and for example, set to a ratio of the first counting result relative to the second counting result ((first counting result)/(second counting result)) such as 1.4, 1.5, and 1.6.

More specifically, in the present embodiment, an aspect (display mode) for displaying the event in the predetermined period on the predetermined display device by type and in association with the monitored person Ob includes a personal history display mode in which a detection history of events detected by the sensor device SU is individually displayed for each person and a personal analysis display mode in which the events of the plurality of monitored persons Ob detected by the sensor device SU are displayed as counting the events for each person and by type of the event. The personal history display mode further includes a time chart display mode in which the detection history of the events detected by the sensor device SU is displayed in a time chart for each day and a graph display mode in which the events detected by the sensor device SU are counted for each counting period and by type of the event and displayed.

In the personal history display mode, the personal history request communication signal is used. The personal history request communication signal stores an instruction (first instruction, first order, first command) for requesting the personal history of the event detected by the sensor device SU, the monitored person's name of the predetermined monitored person Ob which has been input and designated (or sensor ID of sensor device SU for monitoring the predetermined monitored person Ob), the input and designated predetermined period, the display mode, and the like and is transmitted from the terminal devices SP and TA to the management server device SV. The personal history request communication signal stores the time chart display mode or the graph display mode as the display mode.

In the time chart display mode, the monitoring processing part 222 displays a predetermined mark indicating the type of the event on a time axis, which is provided in correspondence with the monitored person Ob, according to the predetermined period at the detection time or the reception time of the event, displays the predetermined mark in a display mode which is different according to whether the event is notified, and displays the night sleeping time and the daytime sleeping time in different display modes so as to make the predetermined display device display the events in the predetermined period by type and in association with the monitored person Ob on the basis of the extraction result of the history information extraction processing part 223. More specifically, the monitoring processing part 222 stores screen data written in a predetermined markup language (electronic file, electronic file of time chart personal history display screen) in a communication signal (history information notification communication signal in time chart display) and transmits the communication signal to the predetermined display device. With the screen data, the predetermined mark indicating the type of the event is displayed on a time axis, which is provided in corresponding to the monitored person Ob, according to the predetermined period at the detection time or the reception time of the event, the predetermined mark is displayed in the display mode which is different according to whether the event is given in notification, and the night sleeping time and the daytime sleeping time are displayed as bars in different display modes. The predetermined mark (symbol, reference symbol, code, and icon) is a graphic mark, for example, a circle and a polygon, and for example, when the event is given in notification, the contour line is indicated by a solid line, and when the event is not given in notification, the contour line is indicated by a broken line. Furthermore, for example, the display color of the contour line may be different according to whether the event is given in notification.

In the graph display mode, the monitoring processing part 222 displays each counting result by type obtained by counting the events corresponding to the monitored person Ob for each counting period and by type in the predetermined period in each field (each row and column) provided in correspondence with the monitored person Ob and provided for each counting period according to the predetermined period as a bar graph (each bar for each column or row of event) in which the events are aligned in order of the type. Accordingly, the monitoring processing part 222 makes the predetermined display device display the events in the predetermined period by type and in association with the monitored person Ob on the basis of the extraction result of the history information extraction processing part 223. More specifically, the monitoring processing part 222 stores screen data written in a predetermined markup language (electronic file, electronic file of graph personal history display screen) in a communication signal (history information notification communication signal in graph display) and transmits the communication signal to the predetermined display device. With the screen data, each counting result by type obtained by counting the events corresponding to the monitored person Ob for each counting period and by type in the predetermined period is displayed in each field provided in correspondence with the monitored person Ob and provided for each counting period according to the predetermined period as a bar graph in which the events are aligned in order of the type of the event. In the graph display mode, the analysis processing part 224 executes the second analysis processing in which the counting period is the sub-period, and it is possible that the monitoring processing part 222 makes the predetermined display device display the comparison result by type which changes by equal to or more than the predetermined threshold obtained by executing the second analysis processing and the comparison result by type which does not change by equal to or more than the predetermined threshold obtained by executing the second analysis processing in different display modes. In this case, the predetermined period stored in the personal history request communication signal is used as the predetermined second period used by the analysis processing part 224.

In the personal analysis display mode, the personal analysis request communication signal is used. The personal analysis request communication signal stores an instruction (second instruction, second order, and second command) for requesting personal analysis of the event detected by the sensor device SU, each monitored person's name of each of the plurality of monitored persons Ob which has been input and designated (or sensor ID of sensor device SU for monitoring each of the plurality of monitored persons Ob), the input and designated predetermined period, and the like and is transmitted from the terminal devices SP and TA to the management server device SV. The number of monitored person's names of the predetermined monitored persons Ob stored in the personal analysis request communication signal may be plural, and all the monitored persons Ob may be collectively designated. In this case, the personal analysis request communication signal stores information (all people collectively designated information) indicating that all the monitored persons Ob are collectively designated as the plurality of monitored person's names.

In the personal analysis display mode, the monitoring processing part 222 displays each counting result by type obtained by counting the events corresponding to the monitored person Ob by type in the predetermined period (predetermined second period) (each first counting result) in each field (row or column) provided for each monitored person Ob as a bar graph (each bar graph for each column or row of event) in which the events are aligned in order of the type. Accordingly, the monitoring processing part 222 makes the predetermined display device display the events in the predetermined period by type and in association with the monitored person Ob on the basis of the extraction result of the history information extraction processing part 223. More specifically, the monitoring processing part 222 stores screen data written in a predetermined markup language (electronic file, electronic file of personal analysis display screen) in a communication signal (analysis information notification communication signal) and transmits the communication signal to the predetermined display device. With the screen data, the monitoring processing part 222 displays each counting result by type obtained by counting the events corresponding to the monitored person Ob in the predetermined period by type in each field provided for each monitored person Ob as a bar graph in which the bars are aligned in order of the type of the event. In the personal analysis display mode, the analysis processing part 224 executes the second analysis processing on each of the plurality of monitored persons Ob, and the monitoring processing part 222 makes the predetermined display device display the comparison result by type which changes by equal to or more than the predetermined threshold obtained by executing the second analysis processing and the comparison result by type which does not change by equal to or more than the predetermined threshold obtained by executing the second analysis processing in different display modes.

The clock part 225 measures year, month, day, hour, and minute.

As indicated by a broken line in Fig. 4, as necessary, the management server device SV may include a server input unit (SV input unit) 24 which is connected to the SV control processing unit 22 and inputs, for example, various commands and various data, a server output unit (SV output unit) 25 which outputs various commands and various data input by the SV input unit 24, monitoring information regarding monitoring of the monitored person Ob, and the like, a server interface unit (SV IF unit) 26 which inputs/outputs data to/from external devices, and the like.

Such a management server device SV can include, for example, a computer having a communication function.

The fixed terminal device SP has a communication function for communicating with the other devices SU, SV, and TA via the network NW, a display function for displaying predetermined information, an input function for inputting a predetermined instruction and data, and the like, and functions as a user interface (UI) of the monitored-person monitoring system MS by inputting the predetermined instruction and data to be applied to the management server device SV and the mobile terminal device TA and displaying the monitoring information obtained by sensor device SU. The fixed terminal device SP includes, for example, a communication interface unit which performs communication, an input unit which inputs various commands, various data, and the like, an output unit which outputs various commands and various data input by the input unit, the monitoring information regarding monitoring of the monitored person Ob, and the like, an interface unit which inputs/outputs data to/from an external device, a storage unit which stores predetermined various programs and predetermined various data, and a control processing unit which controls the communication interface unit, the input unit, the output unit, the interface unit, and the storage unit according to the functions of these units. Such a fixed terminal device SP can include, for example, a computer having a communication function.

For example, a user such as a surveillant inputs permission/rejection of the notification of the event regarding the monitored person Ob by type of the event to the fixed terminal device SP at an appropriate timing such as when the monitored person Ob moves to the facility and the nursing care level of the monitored person Ob changes. When receiving the input operation, the fixed terminal device SP transmits whether the event can be given in notification to the monitored person Ob according to the type of the event to the management server device SV and makes the management server device SV store the transmitted information. As a result, permission/rejection of the notification of the event detected by the sensor device SU regarding the monitored person Ob is set to the management server device SV.

Furthermore, for example, in a case where the user desires to see the personal history of the events detected by the sensor device SU regarding the monitored person Ob, the user inputs the first instruction for requesting the personal history of the events detected by the sensor device SU, a monitored person's name of the target of the personal history to be requested, a target period (the predetermined period) of the personal history to be requested, and a display mode of the personal history to be requested to the fixed terminal device SP. When receiving the input operation, the fixed terminal device SP transmits the personal history request communication signal for storing the above input information to the management server device SV, and when receiving a reply, the fixed terminal device SP displays the returned information.

Furthermore, for example, in a case where the user desires the personal analysis of the events detected by the sensor device SU regarding the monitored person Ob, the user inputs the second instruction for requesting personal analysis of the events detected by the sensor device SU, a plurality of monitored person's names of the target of the personal analysis to be requested, and a target period (the predetermined period) of the personal analysis to be requested to the fixed terminal device SP. When receiving the input operation, the fixed terminal device SP transmits the personal analysis request communication signal for storing the above input information to the management server device SV, and when receiving a reply, the fixed terminal device SP displays the returned information.

The mobile terminal device TA has a communication function for communicating with the other devices SV, SP, and SU via the network NW, a display function for displaying predetermined information, an input function for inputting a predetermined instruction and data, a call function for performing audio communication, and the like. The mobile terminal devices TA inputs the predetermined instruction and data to be applied to the management server device SV and the sensor device SU, displays the monitoring information (including moving image) obtained by the sensor device SU by the notification from the management server device SV, and responds to the nurse call and talks to the monitored person via the audio communication with the sensor device SU. Such a mobile terminal device TA can include, for example, a portable communication terminal device such as a so-called tablet-type computer, smartphone, and a mobile telephone.

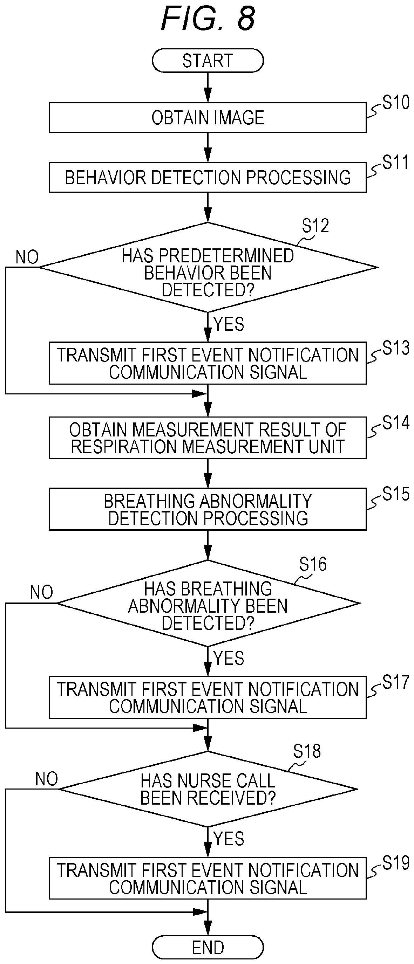

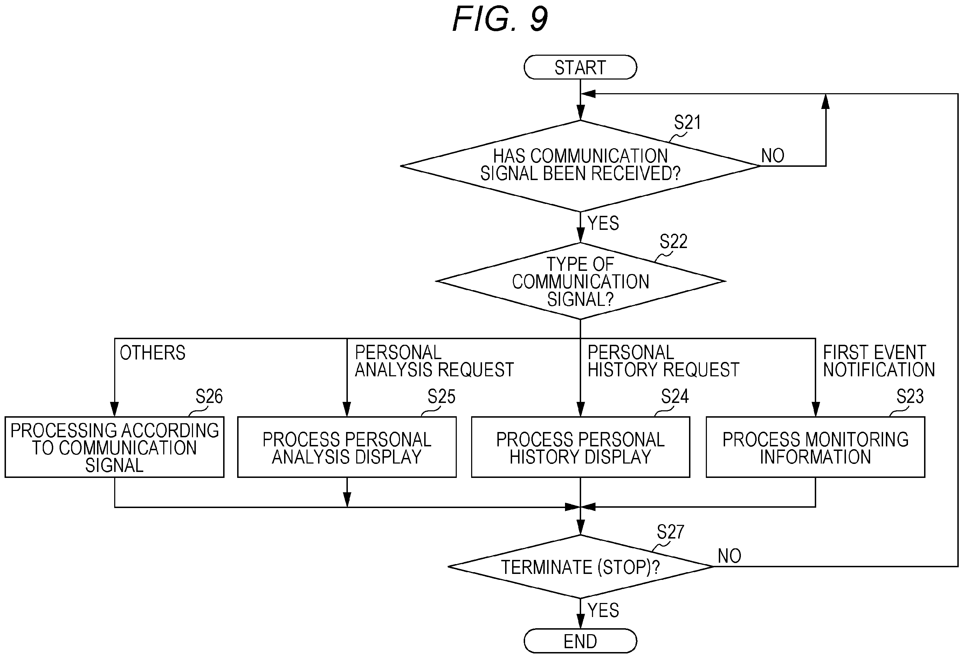

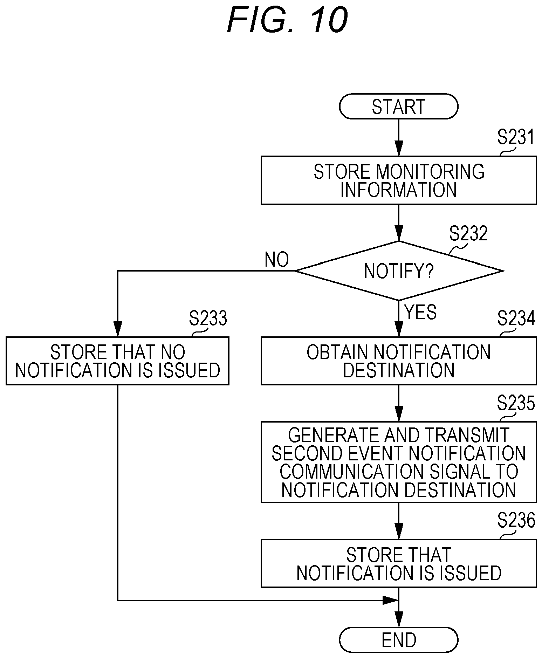

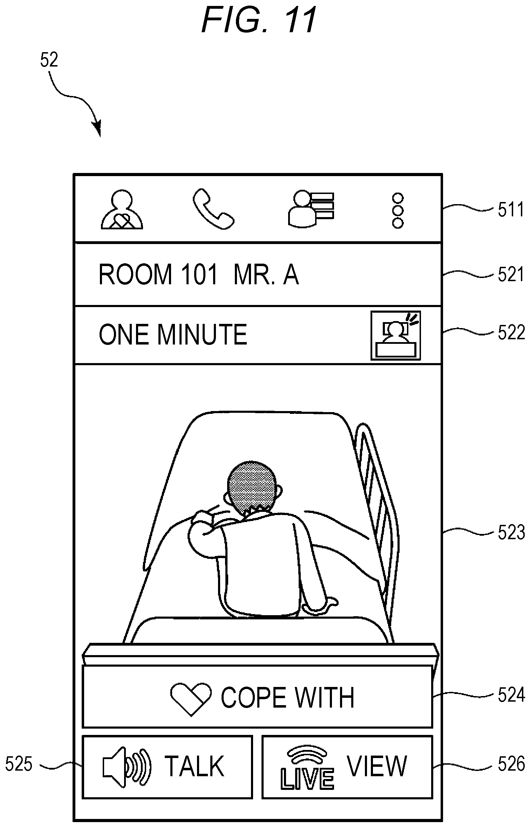

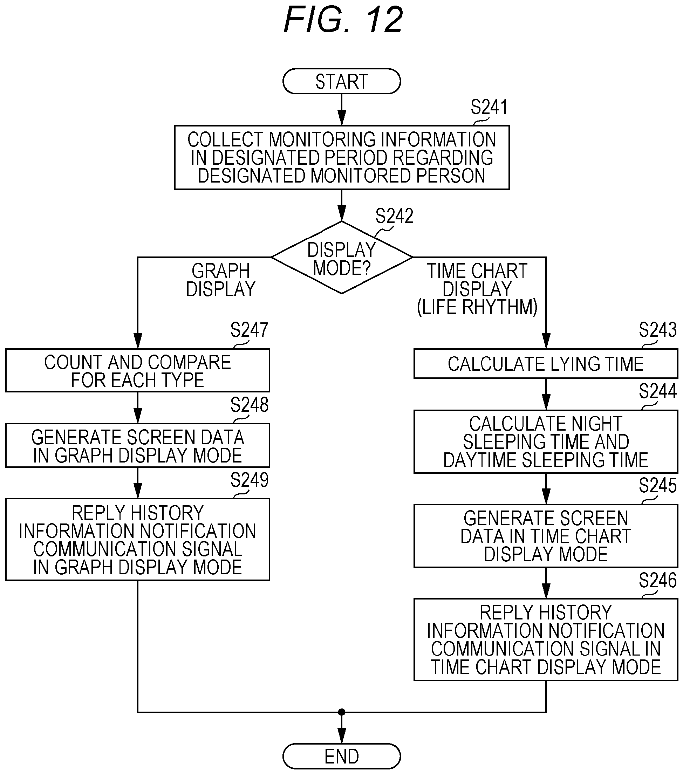

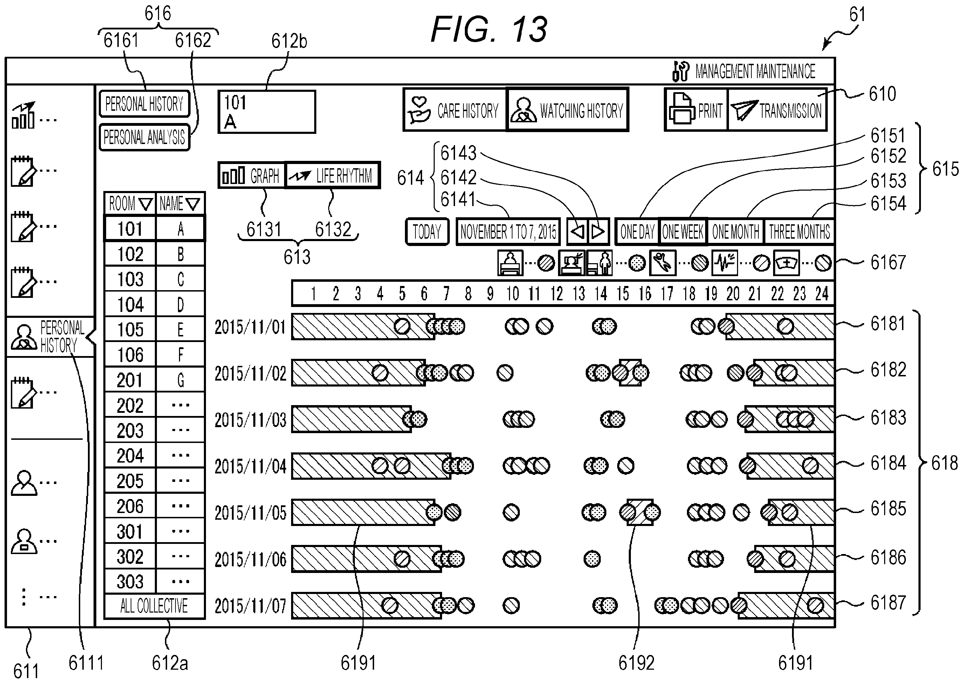

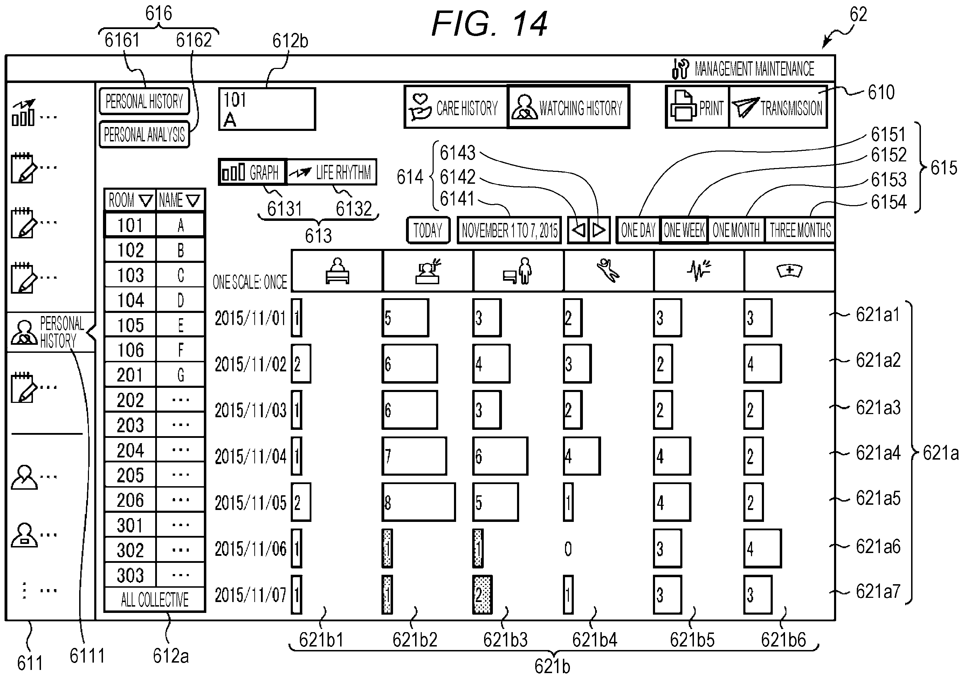

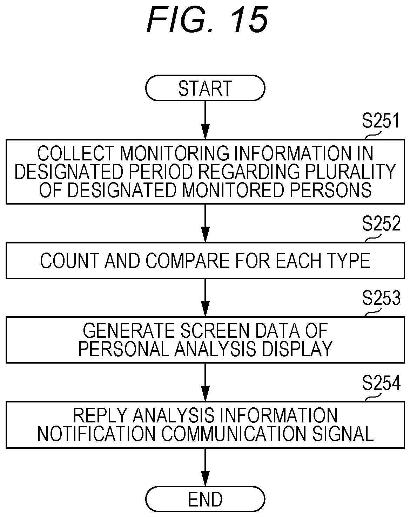

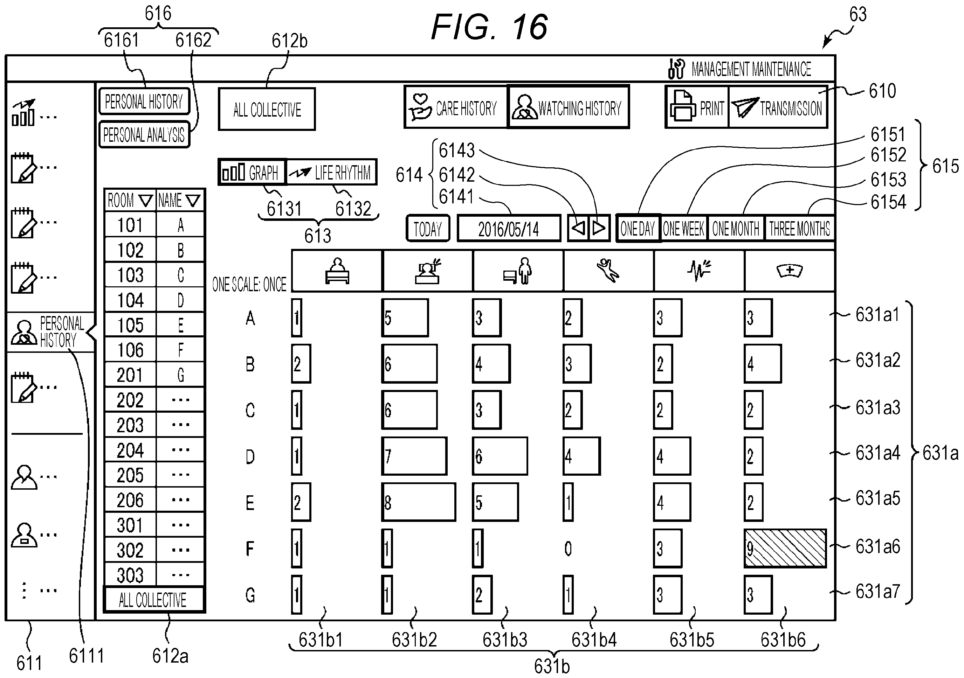

Next, an operation according to the present embodiment will be described. Fig. 8 is a flowchart of an operation of the sensor device illustrated in Fig. 2. Fig. 9 is a flowchart of an operation of the management server device illustrated in Fig. 4. Fig. 10 is a flowchart of processing on the monitoring information in the flowchart illustrated in Fig. 9. Fig. 11 is a diagram of an example of a monitoring information screen displayed on the mobile terminal device illustrated in Fig. 1. Fig. 12 is a flowchart of processing for displaying the personal history in the flowchart in Fig. 9. Fig. 13 is a diagram of an example of a personal history display screen in the time chart displayed on the fixed terminal device illustrated in Fig. 1. Fig. 14 is a diagram of an example of a personal history display screen in the graph displayed on the fixed terminal device illustrated in Fig. 1. Fig. 15 is a flowchart of processing for displaying personal analysis in the flowchart in Fig. 9. Fig. 16 is a diagram of an example of a personal analysis display screen displayed on the fixed terminal device illustrated in Fig. 1.

In the monitored-person monitoring system MS having the above structure, each of the devices SU, SV, SP, and TA initializes and operates necessary units when being turned on. In the sensor device SU, by executing the control processing program, the SU control processing unit 14 functionally includes the SU control unit 141, the behavior detection processing unit 142, the breathing abnormality determination unit 143, the nurse call processing unit 144, and the streaming processing unit 145. In the management server device SV, by executing the control processing program, the SV control processing unit 21 functionally includes the SV control part 211, the monitoring processing part 222, the history information extraction processing part 223, the analysis processing part 224, and the clock part 225.

First, an operation of the sensor device SU will be described. The sensor device SU detects a predetermine behavior of the monitored person Ob, detects the breathing abnormality, and determines whether a nurse call has been received by operating as follows at predetermined sampling intervals, for example, for each frame or for every several frames.

In Fig. 8, first, the sensor device SU obtains an image (image data) for one frame from the imaging unit 111 of the sensor unit 11 by the SU control unit 141 of the SU control processing unit 14 as the target image (S10).

Next, the sensor device SU executes behavior detection processing for detecting a predetermined behavior of the monitored person Ob by the behavior detection processing unit 142 of the SU control processing unit 14 on the basis of the target image obtained in the processing S10 (S11). More specifically, the behavior detection processing unit 142 determines whether the monitored person Ob gets into the bed, gets up, leaves the bed, and falls down.

Next, the sensor device SU determines whether the predetermined behavior of the monitored person Ob has been detected in the behavior detection processing S11 by the behavior detection processing unit 142. As a result of the determination, in a case where the predetermined behavior has not been detected (No), the sensor device SU executes processing S14 next. On the other hand, in a case where the predetermined behavior has been detected (Yes), the sensor device SU executes the processing S14 after executing the next processing S13.

In the processing S13, to notify the predetermined terminal devices SP and TA of the predetermined behavior detected in the processing S11 and S12 via the management server device SV, the sensor device SU transmits the first event notification communication signal regarding the detection of the predetermined behavior as the predetermined event to the management server device SV by the behavior detection processing unit 142. More specifically, the behavior detection processing unit 142 transmits the first event notification communication signal which stores the sensor ID of the system, the predetermined event information (here, one or a plurality of behaviors, i.e., to get into bed, to get up, to leave bed, and to fall down), and the target image related to the event to the management server device SV via the SU communication IF unit 15.

In the processing S14, the sensor device SU obtains the measurement result of the respiration measurement unit 112 of the sensor unit 11 by the SU control unit 141 of the SU control processing unit 14 and stores the result to the SU storage unit 16. The SU storage unit 16 stores at least the measurement result of the respiration measurement unit 112 necessary for the detection of the breathing abnormality of the monitored person Ob.

Next, the sensor device SU executes breathing abnormality detection processing for detecting the breathing abnormality of the monitored person Ob on the basis of the measurement result of the respiration measurement unit 112 by the breathing abnormality determination unit 143 of the SU control processing unit 14 on the basis of the measurement result of the respiration measurement unit 112 obtained in the processing S14 (S15). More specifically, the breathing abnormality determination unit 143 determines whether the following respiration is performed, such as cessation of breathing, sleep apnea, slow respiration, fast respiration, infrequent respiration, hyperventilation, Kussmaul breathing, and Cheyne-Stokes breathing.

Next, the sensor device SU determines whether breathing abnormality of the monitored person Ob has been detected in the breathing abnormality detection processing S15 by the breathing abnormality determination unit 143. As a result of the determination, in a case where the breathing abnormality has not been detected (No), the sensor device SU executes processing S18 next. On the other hand, in a case where the breathing abnormality has been detected (Yes), the sensor device SU executes the processing S18 after executing the next processing S17.

In the processing S17, to notify the predetermined terminal devices SP and TA of the breathing abnormality detected in the processing S14 and S15 via the management server device SV, the sensor device SU transmits the first event notification communication signal regarding the detection of the breathing abnormality as the predetermined event to the management server device SV by the breathing abnormality determination unit 143. More specifically, the breathing abnormality determination unit 143 transmits the first event notification communication signal which stores the sensor ID of the system, the predetermined event information (here, breathing abnormality), and the target image related to the event to the management server device SV via the SU communication IF unit 15.

In the processing S18, the sensor device SU determines whether the nurse call has been received, by the nurse call processing unit 144. That is, the processing S10 to S19 illustrated in Fig. 8 is repeatedly executed at the predetermined sampling intervals. However, it is determined whether the nurse call reception operation unit 13 is operated between the previous execution of the processing S18 and the execution of the processing S18 this time. As a result of the determination, in a case where the nurse call reception operation unit 13 has not been operated and has not received the nurse call (No), the sensor device SU terminates the processing at this time. On the other hand, in a case where the nurse call reception operation unit 13 has been operated and has received the nurse call (Yes), the sensor device SU terminates the processing at this time after executing the next processing S19.

In the processing S19, in order to notify the predetermined terminal devices SP and TA of the nurse call of which the reception has been determined in the processing S18 via the management server device SV, the sensor device SU transmits the first event notification communication signal regarding the nurse call to the management server device SV by the nurse call processing unit 144. More specifically, the nurse call processing unit 144 transmits the first event notification communication signal which stores the sensor ID of the system and the predetermined event information (here, nurse call) to the management server device SV via the SU communication IF unit 15.

Regarding the detection of the predetermined behavior of the monitored person Ob, the detection of the breathing abnormality, and the detection of the reception of the nurse call, the sensor device SU operates as described above.

Next, an operation of the management server device SV will be described. In Fig. 9, the management server device SV determines whether the SV communication IF unit 21 has received a communication signal, by the SV control part 221 of the SV control processing unit 22 (S21). In a case where the communication signal has not been received according to the result of the determination (No), the management server device SV returns to the processing S21. In a case where the communication signal has been received according to the result of the determination (Yes), the management server device SV executes the next processing S22. That is, the management server device SV waits for the reception of the communication signal.

In the processing S22, the management server device SV determines a type of the received communication signal by the SV control part 221. According to the result of the determination, in a case where the received communication signal is the first event notification communication signal (first event notification), the management server device SV executes processing S27 after executing processing S23. In a case where the received communication signal is a personal history request communication signal (personal history request), the management server device SV executes the processing S27 after executing processing S24. In a case where the received communication signal is a personal analysis request communication signal (personal analysis request), the management server device SV executes the processing S27 after executing processing S25. In a case where the received communication signal is not any one of the first event notification communication signal, the personal history request communication signal, and the personal analysis request communication signal (other), the management server device SV executes the processing S27 after executing processing S26.

In the processing S23, the management server device SV processes the first event notification communication signal received from the sensor device SU in the processing S21, by the monitoring processing part 222 of the SV control processing unit 22.

More specifically, in Fig. 10, first, the monitoring processing part 222 stores (record) the monitoring information regarding monitoring of the monitored person Ob stored in the first event notification communication signal received in the processing S21 in the monitoring information storage part 231 (S231). More specifically, the monitoring processing part 222 creates a new record in the monitoring information table MT stored in the monitoring information storage part 231, registers the sensor ID and the event information stored in the first event notification communication signal received in the processing S21 respectively in a sensor ID field 2311 and an event field 2312 in the new record, obtains year, month, day, hour, and minute from the clock part 225, registers the obtained year, month, day, hour, and minute in a reception time field 2313 of the new record, registers a target image (here, file name of target image) in a still image field 2314 of the new record in a case where the target image is stored in the first event notification communication signal received in the processing S21, searches a communication address corresponding to the sensor ID stored in the first event notification communication signal received in the processing S21 from the communication address correspondence information table DT stored in the inter-device information storage part 232, registers the searched communication address in a moving image field 2315 of the new record, and registers a notification flag "0" and a coping flag "0" which are default values in a notification field 2316 and a coping field 2317 of the new record respectively.

Next, the monitoring processing part 222 determines whether to notify the predetermined terminal devices SP and TA of the first event notification communication signal received in the processing S21 by a second event notification communication signal. More specifically, the monitoring processing part 222 selects (searches) the record, in which the sensor ID stored in the first event notification communication signal received in the processing S21 is registered in the sensor ID field 2321, from the notification destination correspondence information table AT stored in the inter-device information storage part 232 and obtains notification permission/rejection flags registered in the subfields 23221 to 23226 corresponding to the event information stored in the first event notification communication signal received in the processing S21 in the notification permission/rejection field 2322 which the selected record. For example, in a case where the first event notification communication signal received in the processing S21 stores "SU-1" as a sensor ID and "to leave the bed" as the event information, the notification permission/rejection flag "1" is obtained from the notification destination correspondence information table AT illustrated in Fig. 6A. In a case where the obtained notification permission/rejection flag is "0", the monitoring processing part 222 determines that this is a case where the first event notification communication signal received in the processing S21 is not given in notification to the predetermined terminal devices SP and TA by the second event notification communication signal (No), and after subsequently executing the processing S233, the monitoring processing part 222 terminates the present processing S23. In a case where the obtained notification permission/rejection flag is "1", the monitoring processing part 222 determines that this is a case where the first event notification communication signal received in the processing S21 is given in notification to the predetermined terminal devices SP and TA by the second event notification communication signal (Yes), after sequentially executing processing S234, S235, and S236, the monitoring processing part 222 terminates the present processing S23.

In this processing S233, the monitoring processing part 222 registers the notification flag "0" which means no notification in the notification field 2316 in the record which has been newly generated in the monitoring information table MT in the processing S231 and terminates the present processing S23. As described above, the notification flag "0" is registered in the notification field 2316 as a default value, the processing S233 may be omitted. However, by executing the processing S233, the fact caused by the first event notification communication signal that the second event notification communication signal is not given in notification is surely recorded (stored) in the monitoring information storage part 231.

In the processing S234, the monitoring processing part 222 selects and obtains a notification destination (re-notification destination, transfer destination, and transmission destination) corresponding to the sensor device SU which has transmitted the first event notification communication signal received in the processing S21 from the notification destination relationship stored in the inter-device information storage part 232. More specifically, the monitoring processing part 222 selects (searches) the record, in which the sensor ID stored in the first event notification communication signal received in the processing S21 is registered in the sensor ID field 2321, from the notification destination correspondence information table AT stored in the inter-device information storage part 232 and obtains a terminal ID registered in the notification destination field 2323 in the selected record. Then, the monitoring processing part 222 selects (searches) the record in which the obtained terminal ID is registered in the ID field 2325 from the communication address correspondence information table DT stored in the inter-device information storage part 232 and obtains a communication address registered in a communication address field 2326 in the selected record.

Subsequent to the processing S234, in the processing S235, the monitoring processing part 222 generates a second event notification communication signal corresponding to the first event notification communication signal received in the processing S21 and transmits the generated second event notification communication signal to the notification destination obtained in the processing S234

Subsequent to the processing S235, in the processing S236, the monitoring processing part 222 registers the notification flag "1", which means that the notification is issued, in the notification field 2316 in the record which has been newly generated in the monitoring information table MT in the processing S231 and terminates the present processing S23.

In this way, the first event notification communication signal received from the sensor device SU is processed in the processing S21.

Here, the terminal devices SP and TAwhich have received the second event notification communication signal transmitted in the processing S235 display a screen according to each piece of information stored in the received second event notification communication signal. For example, in a case where the second event notification communication signal regarding the predetermined behavior has been received, the terminal devices SP and TA display a monitoring information screen 52 to display each piece of information related to the predetermined behavior.

For example, as illustrated in Fig. 11, the monitoring information screen 52 includes, for example, a menu bar region 511 for displaying a menu bar is displayed, a monitored person's name region 521 for displaying a disposed place of the sensor device SU having the sensor ID stored in the received second event notification communication signal and the name of the monitored person Ob to be monitored by the sensor device SU having the sensor ID, a detection information display region 522 for displaying an elapsed time from the reception time of the received second event notification communication signal (or detection time of the predetermined behavior) and the event information stored in the received second event notification communication signal (detection result of the predetermined behavior), an image region 523 for displaying an image stored in the received second event notification communication signal (that is, target image imaged by the sensor device SU having the sensor ID) (here, still image), a "cope with" button 524, a "talk" button 525, and a "view LIVE" button 526.

To display the disposed place of the sensor device SU and the name of the monitored person Ob in the monitored person's name region 521, the sensor ID, the disposed place of the sensor device SU having the sensor ID, and the name of the monitored person Ob to be monitored by the sensor device SU having the sensor ID are previously stored in the terminal devices SP and TA in association with each other. In the detection information display region 522, the detection result stored in the received second event notification communication signal (in the present embodiment, each name of to get into bed, to get up, to leave bed, to fall down, and breathing abnormality) may be displayed as it is. However, in the present embodiment, an icon symbolically indicating the detection result is displayed. To display the icon, each behavior and the icon which symbolically indicates the behavior are stored in advance in the terminal devices SP and TA in association with each other. In the example illustrated in Fig. 11, a get-up icon which symbolically indicates to get up is displayed in the detection information display region 522. The "cope with" button 524 is a button to input implementation intention information which indicates that a user of the terminal devices SP and TA has an intention to make predetermined countermeasure (cope with, treat, measure), for example, life saving, nursing, nursing care, and assistance relative to the detection result displayed on the monitoring information screen 52 to the terminal devices SP and TAin the monitoring information screen 52. The "talk" button 525 is a button to request audio communication and to input an instruction to communicably connect the sensor device SU having the sensor ID to the terminal devices SP and TAvia the network NW. The "view LIVE" button 526 is a button to request a live moving image and to input an instruction to display a moving image imaged by the sensor device SU having the sensor ID.

Returning to Fig. 9, in the processing S24, the management server device SV processes the personal history request communication signal received from the terminal devices SP and TA in the processing S21 by the SV control processing unit 22.

More specifically, in Fig. 12, first, the SV control processing unit 22 collects the monitoring information in the predetermined period designated by the personal history request communication signal regarding the monitored person Ob designated by the personal history request communication signal received in the processing 21 from the monitoring information storage part 321 by the history information extraction processing part 223 (S241). More specifically, the history information extraction processing part 223 searches the sensor ID corresponding to the monitored person's name of the monitored person Ob stored in the personal history request communication signal received in the processing S21 from the sensor information stored in the sensor information storage part 233. In a case where the personal history request communication signal stores the sensor ID instead of or in addition to the monitored person's name of the monitored person Ob, this processing can be omitted. Then, the history information extraction processing part 223 searches and extracts the event (detection result) which is in the predetermined period stored in the personal history request communication signal and is corresponding to the searched sensor ID, the reception time, and whether the event has been given in notification from the monitoring information stored in the monitoring information storage part 231. The history information extraction processing part 223 outputs the monitored person's name of the monitored person Ob and the predetermined period stored in the personal history request communication signal, a pair of or a plurality of pairs of extracted events (detection result), the reception time, and whether the event has been given in notification to the monitoring processing part 222.

Next, the SV control processing unit 22 determines the display mode stored in the personal history request communication signal received in the processing S21 by the monitoring processing part 222 (S242). According to the result of the determination, in a case where the display mode is the time chart display mode (time chart display (life rhythm)), the monitoring processing part 222 sequentially executes processing S243, S244, S245, and S246, and then, terminates the present processing S24. On the other hand, according to the result of the determination, in a case where the display mode is the graph display mode (graph display), the monitoring processing part 222 sequentially executes processing S247, S248, and S249, and then, terminates the present processing S24.

In this processing S243, the SV control processing unit 22 obtains a time from a time to get into the bed to a time to leave the bed as a lying time on the basis of the extraction result of the history information extraction processing part 223 in the processing S241 by the analysis processing part 224. More specifically, the analysis processing part 224 extracts all the pairs of getting into the bed and leaving the bed sequentially arranged adjacent to each other in time-series manner by referring to the reception times from the monitoring information collected in the processing S241 and subtracts the reception time of getting into the bed from the reception time of leaving the bed for each pair so as to obtain the lying time of each pair ((lying time) = (reception time of leaving the bed) - (reception time of getting into the bed)).

Subsequent to the processing S243, in the processing S244, the SV control processing unit 22 obtains a time from the first time to get into the bed on or after the predetermined first time (for example, 20 o'clock) to the first time to leave the bed on or after the predetermined second time on the next day (for example, five o'clock) as a sleeping time (night sleeping time) from the lying times of the pairs arranged in the time-series manner obtained in the processing S243 by the analysis processing part 224 and obtains the lying time excluding the obtained sleeping time as a daytime sleeping time. In a case where once or a plurality of times to leave the bed and to get into the bed are detected between the first time to get into the bed on or after the predetermined first time and the first time to leave the bed on or after the predetermined second time on the next day, the lying times between the first time to get into the bed on or after the predetermined first time and the first time to leave the bed on or after the predetermined second time on the next day are added as the sleeping time (night sleeping time).

Subsequent to the processing S244, in the processing S245, the SV control processing unit 22 generates screen data (electronic file) of the time chart personal history display screen on the basis of the extraction result of the history information extraction processing part 223 by the processing S241 and the analysis result of the analysis processing part 224 by the processing S243 and S244 by the monitoring processing part 222. More specifically, the SV control processing unit 22 generates the screen data (electronic file) of the time chart personal history display screen written in a predetermined markup language in which a predetermined mark indicating the type of the event is displayed on the time axis, which is provided in correspondence with the monitored person Ob, according to the predetermined period at the reception time of the event on the basis of the extraction result and the analysis result, the predetermined mark is displayed as bars in the different display modes according to whether the event has been given in notification, and the night sleeping time and the daytime sleeping time are displayed as bars in display modes different from each other.

More specifically, for example, in a case where a time chart personal history display screen 61 is displayed on the terminal devices SP and TA (here, fixed terminal device SP), as illustrated in Fig. 13, the time chart personal history display screen 61 includes a menu region 611, a monitored-person menu region 612a, a monitored person's name display region 612b, a mode menu region 613, a period input region 614, a period length menu region 615, a personal history analysis menu region 616, an example display region 617, a time chart personal history display region 618, and a "transmission" button 610.

The menu region 611 is a region for displaying one or a plurality of selectable menu buttons, and a "personal history" button 6111 is included in the one or the plurality of menu buttons. The "personal history" button 6111 is a button for inputting an instruction to display the events in the predetermined period by type and in association with the monitored person Ob. When receiving an input operation of the "personal history" button 6111, the terminal devices SP and TA display a screen of a display mode which has been displayed last time, in the present embodiment, the time chart personal history display screen 61 illustrated in Fig. 13, a graph personal history display screen 62 illustrated in Fig. 14 to be described later, or a personal analysis display screen 63 illustrated in Fig. 16 to be described later.

The monitored-person menu region 612a is a region for displaying a list of one or a plurality of selectable monitored persons Ob. In the example illustrated in Fig. 13, in the monitored-person menu region 612a, sets (pairs) of rooms where the monitored persons Ob enter (for example, room number, in other words, disposed place of sensor device SU provided in correspondence with monitored person Ob) and the monitored person's names are sorted in a descending order with a predetermined criteria and are displayed in a list. The criterion of the sort is arbitrary and, for example, Japanese alphabetical order of the monitored person's names (alphabetical order) and a room number order. In the example illustrated in Fig. 13, the sets of the monitored person's names and the room names are sorted in the room number order. Individual regions where the sets of the monitored person's name and the room name are respectively displayed are buttons to input a monitored person Ob selected by a surveillant (user) to the terminal devices SP and TA (monitored-person selection button). In addition, in the present embodiment, the monitored-person menu region 612a includes an "all collective" button used to collectively select one or all of the plurality of selectable monitored persons Ob and input all the selected monitored persons Ob to the terminal devices SP and TA.

The monitored person's name display region 612b is a region selected in the monitored-person menu region 612a and displays the name of the monitored person Ob corresponding to the information displayed on the screens 61, 62, and 63 (including all the monitored persons).

The mode menu region 613 is a region for displaying a plurality of selectable mode buttons. In the example illustrated in Fig. 13, the mode menu region 613 includes a "graph" button 6131 to input an instruction to designate the graph display mode and a "life rhythm" button 6132 to input an instruction to designate the time chart display mode.

The period input region 614 is a region to input the predetermined period. In the example illustrated in Fig. 13, the period input region 614 includes a year/month/day input region 6141 to directly input the predetermined period with year, month, and day, a year/month/day backward button 6142 which temporally moves a date displayed in the year/month/day input region 6141 backward day by day, and a year/month/day forward button 6143 which temporally moves the date displayed in the year/month/day input region 6141 forward day by day.

The period length menu region 615 is a region to input the period length of the predetermined period input in the period input region 614. In the example illustrated in Fig. 13, the period length menu region 615 includes a "one day" button 6151 to set the period length to one day and input, a "one week" button 6152 to set the period length to one week and input, a "one month" button 6153 to set the period length to one month and input, and a "three months" button 6154 to set the period length to three months and input.

The personal history analysis menu region 616 is a region for displaying the plurality of selectable display mode buttons. In the example illustrated in Fig. 13, the personal history analysis menu region 616 includes a "personal history" button 6161 to input an instruction to designate the personal history display mode and a "personal analysis" button 6162 to input an instruction to designate the personal analysis display mode.

The example display region 617 is a region for displaying an example of the predetermined mark which is displayed in the time chart personal history display region 618 and indicates the type of the event. In the example illustrated in Fig. 13, the predetermined mark includes a first mark representing getting into the bed detected and given in notification by the sensor device SU, a second mark representing getting up and leaving the bed detected and given in notification by the sensor device SU, a third mark representing falling down detected and given in notification by the sensor device SU, a fourth mark representing the breathing abnormality detected and given in notification by the sensor device SU, and a fifth mark representing the nurse call detected and given in notification by the sensor device SU.

The time chart personal history display region 618 is a region where the events in the predetermined period are displayed by type and in association with the monitored person Ob and a region for displaying the detection history of the events detected by the sensor device SU in a time chart for each day in the time chart personal history display screen 61. More specifically, the predetermined mark indicating the type of the event (first to fifth marks in example illustrated in Fig. 13) is displayed on the time axis according to the predetermined period at the reception time of the event, the predetermined mark is displayed in the display mode which is different according to whether the event has been given in notification, and the night sleeping time and the daytime sleeping time are displayed in the display modes different from each other. In the example illustrated in Fig. 13, to display the personal history for one week in the time chart, the time chart personal history display region 618 includes seven of first to seventh time axis regions 6181 to 6187 for displaying respective time axes of the first to the seventh days in each of rows arranged from the upper side to the lower side. On each of the time axes of the first to seventh time axis regions 6181 to 6187, the predetermined mark according to the event detected by the sensor device SU is displayed at the reception time. For example, on the time axis of the first time axis region 6181, from zero o'clock to 24 o'clock, the following marks are sequentially displayed, i.e., a single fourth mark representing breathing abnormality, four second marks representing getting up and leaving the bed, four fifth marks representing the nurse call, two second marks representing getting up and leaving the bed, three fifth marks representing the nurse call, a single first mark representing getting into the bed, and a single fourth mark representing the breathing abnormality. Each of these 16 marks is indicated by a solid contour line indicating that the event has been given in notification. A broken contour line indicates that the event has not been given in notification. The night sleeping time and the daytime sleeping time are displayed as bars (rectangle) on the respective time axes of the first to seventh time axis region 6181 to 6187, the night sleeping time is displayed as a bar 6191 which is hatched with a plurality of left diagonal lines from the upper left to the lower right, and the daytime sleeping time is displayed by a bar 6192 which is hatched with a plurality of right diagonal lines from the upper right to the lower left. In a case where the number of days to be displayed exceeds the number of days which can be displayed in the time chart personal history display region 618, the time chart personal history display region 618 can be scrolled upward and downward.

The "transmission" button 610 is a button to input an instruction to transmit the input content input on the time chart personal history display screen 61 from the terminal devices SP and TAto the management server device SV and update the time chart personal history display screen 61 to the content returned from the management server device SV according to the transmitted input content. When receiving the input operation of the "transmission" button 610, in a case where the "personal history" button 6161 is input and the "life rhythm" button 6132 is input, the terminal devices SP and TAtransmit the personal history request communication signal, which stores the first instruction for requesting the personal history of the event detected by the sensor device SU, the monitored person's name of the monitored person Ob input and designated in the monitored-person menu region 612a ("K yama M ta" in example illustrated in Fig. 13), the period input and designated in the period input region 614 and the period length menu region 615 (the predetermined period) (seven days from November 1, 2015 to November 7, 2015 in example in Fig. 13), the time chart display mode, and the like, to the management server device SV.

When receiving the personal history request communication signal, the management server device SV sequentially executes the processing S21, S22, and S24, and in the processing S24, the management server device SV executes the processing S241, S242, S243, S244, and S245. In the processing S245, the monitoring processing part 222 generates an electronic file of the time chart personal history display screen 61 illustrated in Fig. 13 by using the predetermined markup language on the basis of the extraction result of the history information extraction processing part 223 by the processing S241 and the analysis result of the analysis processing part 224 by the processing S243 and S244.

Returning to Fig. 12, subsequent to the processing S245, in the processing S246, the control processing unit 22 replies the history information notification communication signal in the time chart display mode for storing the electronic file of the time chart personal history display screen 61 generated in the processing S245 to the terminal devices SP and TA (here, fixed terminal device SP) by the monitoring processing part 222 and terminates the present processing S24. When receiving the history information notification communication signal in the time chart display mode, the terminal devices SP and TA (here, fixed terminal device SP) display (update) the time chart personal history display screen 61 by using the electronic file of the time chart personal history display screen 61 stored in the received history information notification communication signal in the time chart display mode.

On the other hand, in the processing S247, the control processing unit 22 executes the second analysis processing, in which the analysis processing part 224 obtains the first counting result by counting the events corresponding to the predetermined monitored person Ob by type within the predetermined second period which is an analysis target on the basis of the extraction result of the history information extraction processing part 223 by the processing S241, obtains the second counting result by counting the events corresponding to the predetermined monitored person Ob by type in a predetermined third period before the second period and obtaining an average value according to the second period length, compares the first counting result and the second counting result to obtain the comparison result by type which changes by equal to or more than the predetermined threshold. More specifically, the analysis processing part 224 sets the predetermined period stored in the personal history request communication signal received in the processing S21 as the predetermined second period on the basis of the extraction result of the history information extraction processing part 223 by the processing S241 and counts the events by type for each of the plurality of sub-period in the predetermined second period, here, one day as a default value so as to obtain each first counting result for each of the plurality of sub-period (each day). Next, the analysis processing part 224 obtains the second counting result by obtaining an average value per day on the basis of the counting result of counting the events corresponding to the predetermined monitored person Ob by type within the predetermined third period before the predetermined second period, here, for one week which is a default value. Then, the analysis processing part 224 compares the first counting result and the second counting result by type for each of the plurality of sub-periods in the predetermined second period, here, for each day which is a default value and obtains the comparison result by type which changes by equal to or more than the predetermined threshold.

Subsequent to the processing S247, in the processing S248, the SV control processing unit 22 generates the screen data (electronic file) of the graph personal history display screen on the basis of the extraction result of the history information extraction processing part 223 by the processing S241 and the analysis result of the analysis processing part 224 by the processing S247 by the monitoring processing part 222. More specifically, the SV control processing unit 22 generates the screen data (electronic file) of the graph personal history display screen written in a predetermined markup language in which each second counting result by type obtained by counting the events corresponding to the monitored person Ob within the predetermined second period for each sub-period is displayed in each field provided in correspondence with the monitored person Ob and for each sub-period according to the second period as each of bar graphs arranged in order of the type of the event on the basis of the extraction result and the analysis result, and in which the comparison result by type which changes by equal to or more than the predetermined threshold on the basis of the analysis result and the comparison result by type which does not change by equal to or more than the predetermined threshold on the basis of the analysis result are displayed in display modes different from each other.

More specifically, for example, in a case where the graph personal history display screen 62 is displayed on the terminal devices SP and TA (here, fixed terminal device SP), as illustrated in Fig. 14, the graph personal history display screen 62 includes a menu region 611, a monitored-person menu region 612a, a monitored person's name display region 612b, a mode menu region 613, a period input region 614, a period length menu region 615, a personal history analysis menu region 616, graph personal history display regions 621 (621a and 621b), and a "transmission" button 610. Since the menu region 611, the monitored-person menu region 612a, the monitored person's name display region 612b, the mode menu region 613, the period input region 614, the period length menu region 615, the personal history analysis menu region 616, and the "transmission" button 610 in the graph personal history display screen 62 are respectively similar to the menu region 611, the monitored-person menu region 612a, the monitored person's name display region 612b, the mode menu region 613, the period input region 614, the period length menu region 615, the personal history analysis menu region 616, and the "transmission" button 610 in the time chart personal history display screen 61, description thereof will be omitted.

The graph personal history display region 621 (621a and 621b) is a region for displaying the events within the predetermined second period by type and in association with the monitored person Ob and a region for counting and displaying the events detected by the sensor device SU for each sub-period of the counting period and by type of the event in the graph personal history display screen 62. More specifically, each second counting result by type obtained by counting the events corresponding to the monitored person Ob within the predetermined second period for each sub-period and by type is displayed in each field provided for each sub-period according to the predetermined second period as each of the bar graphs arranged in order of the type of the event, and the comparison result by type which changes by equal to or more than the predetermined threshold on the basis of the analysis result and the comparison result by type which does not change by equal to or more than the predetermined threshold on the basis of the analysis result are displayed in the display modes different from each other. In the example illustrated in Fig. 14, to display the counting results per day for one week, the graph personal history display region 621 includes first to seventh graph display regions 621a1 to 621a7 respectively for the first to seventh days in rows arranged from the upper side to the lower side. Then, to display each counting region by type per day by a bar graph, each of the first to seventh graph display regions 621a1 to 621a7 further includes sub-graph regions 621b as many as the number of types of the event. In the present embodiment, the sub-graph region 621b includes a get-into-bed sub-graph region 621b1 which indicates a counting result of the number of times to get into the bed per day by a bar graph, a get-up sub-graph region 621b2 which indicates a counting result of the number of times to get up per day by a bar graph, a leave-from-bed sub-graph region 621b3 which indicates a counting result of the number of times to leave the bed per day by a bar graph, a fall-down sub-graph region 621b4 which indicates a counting result of the number of times to fall down by a bar graph, a breathing abnormality sub-graph region 621b5 which indicates a counting result of the number of times of breathing abnormality per day by a bar graph, and a nurse call sub-graph region 621b6 which indicates a counting result of the number of times of nurse calls per day by a bar graph. For example, in the first graph display region 621a1 of the first day, in the get-into-bed sub-graph region 621b1, the get-up sub-graph region 621b2, the leave-from-bed sub-graph region 621b3, the fall-down sub-graph region 621b4, the breathing abnormality sub-graph region 621b5, and the nurse call sub-graph region 621b6, a bar graph indicating once, a bar graph indicating five times, a bar graph indicating three times, a bar graph indicating twice, a bar graph indicating three times, and a bar graph indicating three times are respectively displayed. The bar graphs displayed in the graph personal history display region 621 are displayed in the display modes different from each other according to the comparison result by type which changes by equal to or more than the predetermined threshold on the basis of the analysis result and the comparison result by type which does not change by equal to or more than the predetermined threshold on the basis of the analysis result. For example, in a case where the comparison result by type changes by equal to or more than the predetermined threshold, the bar graph is indicated by hatching of a plurality of points, and in a case where the comparison result by type does not change by equal to or more than the predetermined threshold, the bar graph is indicated by an outline. In the example illustrated in Fig. 14, the bar graphs displayed in the get-up sub-graph region 621b2 and the leave-from-bed sub-graph region 621b3 in the sixth graph display region 621a6 of the sixth day and the bar graphs displayed in the get-up sub-graph region 621b2 and the leave-from-bed sub-graph region 621b3 in the seventh graph display region 621a7 of the seventh day are hatched by the plurality of points, and this indicates that the comparison result by type changes by equal to or more than the predetermined threshold. In a case where the number of days to be displayed exceeds the number of days which can be displayed in the graph personal history display region 621 (621a and 621b), the graph personal history display region 621 (621a and 621b) can be scrolled upward and downward.

For example, in the time chart personal history display screen 61 illustrated in Fig. 13 or in the graph personal history display screen 62 illustrated in Fig. 14, when the surveillant (user) performs an input operation on the "personal history" button 6161, performs an input operation on the "graph" button 6131, performs an input operation on the monitored-person menu region 612a, performs input operations on the period input region 614 and the period length menu region 615, and performs an input operation on the "transmission" button 610, the terminal devices SP and TA (here, fixed terminal device SP) transmit the personal history request communication signal, which stores the first instruction for requesting the personal history of the event detected by the sensor device SU, the monitored person's name of the monitored person Ob input and designated in the monitored-person menu region 612a ("K yama M ta" in example illustrated in Fig. 14), the periods input and designated in the period input region 614 and the period length menu region 615 (the predetermined period, in other words, here, the predetermined second period) (seven days from November 1, 2015 to November 7, 2015 in example illustrated in Fig. 14), the graph display mode, and the like, to the management server device SV.

When receiving the personal history request communication signal, the management server device SV sequentially executes the processing S21, S22, and S24, and in the processing S24, the management server device SV executes the processing S241, S242, S247, and S248. In the processing S248, the monitoring processing part 222 generates the electronic file of the graph personal history display screen 62 illustrated in Fig. 14 by using the predetermined markup language on the basis of the extraction result of the history information extraction processing part 223 by the processing S241 and the analysis result of the analysis processing part 224 by the processing S247.

Returning to Fig. 12, subsequent to the processing S248, in the processing S249, the control processing unit 22 replies the history information notification communication signal in the graph display mode for storing the electronic file of the graph personal history display screen 62 generated in the processing S248 to the terminal devices SP and TA (here, fixed terminal device SP) by the monitoring processing part 222 and terminates the present processing S24. When receiving the history information notification communication signal in the graph display mode, the terminal devices SP and TA (here, fixed terminal device SP) display (update) the graph personal history display screen 62 by using the electronic file of the graph personal history display screen 62 stored in the received history information notification communication signal in the graph display mode.

In the processing S24, the management server device SV processes the personal history request communication signal received from the terminal devices SP and TAin the processing S21.

Returning to Fig. 9, in the processing S25, the management server device SV processes the personal analysis request communication signal received from the terminal devices SP and TA in the processing S21 by the SV control processing unit 22.

More specifically, in Fig. 15, first, the SV control processing unit 22 collects monitoring information in the predetermined period designated by the personal analysis request communication signal regarding the plurality of monitored persons Ob designated by the personal analysis request communication signal received in the processing 21 from the monitoring information storage part 321 by the history information extraction processing part 223 (S251). More specifically, the history information extraction processing part 223 searches each sensor ID corresponding to each of the monitored person's names of the monitored persons Ob stored in the personal analysis communication signal received in the processing S21 from the sensor information stored in the sensor information storage part 233. In a case where the personal analysis request communication signal stores the sensor ID instead of or in addition to the monitored person's name of the monitored person Ob, this processing can be omitted. In addition, in a case where all the monitored persons Ob are collectively designated, this processing can be omitted. Then, for each searched sensor ID, the history information extraction processing part 223 searches the event (detection result) to be coped with in the predetermined period stored in the personal analysis request communication signal from the monitoring information stored in the monitoring information storage part 231 and extracts the searched event. Then, the history information extraction processing part 223 outputs the monitored person's name of each of the plurality of monitored persons Ob and the predetermined period stored in the personal analysis request communication signal and the plurality of events (detection result) respectively corresponding to the plurality of monitored persons Ob, from which the monitored person's name and the predetermined period have been extracted, to the monitoring processing part 222.

Next, the control processing unit 22 executes the second analysis processing on each of the plurality of monitored persons Ob stored in the personal analysis request communication signal on the basis of the extraction result of the history information extraction processing part 223 by the processing S251 by the analysis processing part 224 (S252). More specifically, the analysis processing part 224 obtains the first counting result by counting the events in the second period by type regarding each of the plurality of monitored persons Ob stored in the personal analysis request communication signal on the basis of the extraction result of the history information extraction processing part 223 by the processing S241 as assuming that the predetermined period stored in the personal analysis request communication signal be the predetermined second period. Next, the analysis processing part 224 obtains the second counting result by obtaining the average value of the second period on the basis of the counting result obtained by counting the events corresponding to the monitored person Ob for the predetermined third period before the second period, here, for one week as a default value by type, for each of the plurality of monitored persons Ob stored in the personal analysis request communication signal. Then, the analysis processing part 224 compares the first counting result and the second counting result by type for each of the plurality of monitored persons Ob stored in the personal analysis request communication signal and obtains the comparison result by type which changes by equal to or more than the predetermined threshold.

Next, the SV control processing unit 22 generates the screen data (electronic file) of the personal analysis display screen on the basis of the extraction result of the history information extraction processing part 223 by the processing S251 and the analysis result of the analysis processing part 224 by the processing S252 by the monitoring processing part 222. More specifically, the SV control processing unit 22 generates the screen data (electronic file) of the personal analysis display screen written in a predetermined markup language in which each counting result (each first counting result) by type obtained by counting the events corresponding to the monitored person Ob by type in the predetermined period (the predetermined second period) is displayed in each field (each row or each column) provided for each monitored person Ob by each of the bar graphs arranged in order of the type of the event (bar graph in each column or row for each event) on the basis of the extraction result and the analysis result, and the comparison result by type which changes by equal to or more than the predetermined threshold on the basis of the analysis result and the comparison result by type which does not change by equal to or more than the predetermined threshold on the basis of the analysis result are displayed in the display modes different from each other.

More specifically, for example, in a case where the personal analysis display screen 63 is displayed on the terminal devices SP and TA (here, fixed terminal device SP), as illustrated in Fig. 16, the personal analysis display screen 63 includes a menu region 611, a monitored-person menu region 612a, a monitored person's name display region 612b, a mode menu region 613, a period input region 614, a period length menu region 615, a personal history analysis menu region 616, personal analysis display regions 631 (631a and 631b), and the "transmission" button 610. Since the menu region 611, the monitored-person menu region 612a, the monitored person's name display region 612b, the mode menu region 613, the period input region 614, the period length menu region 615, the personal history analysis menu region 616, and the "transmission" button 610 in the personal analysis display screen 63 are respectively similar to the menu region 611, the monitored-person menu region 612a, the monitored person's name display region 612b, the mode menu region 613, the period input region 614, the period length menu region 615, the personal history analysis menu region 616, and the "transmission" button 610 in the time chart personal history display screen 61, description thereof will be omitted.

The personal analysis display region 631 (631a and 631b) is a region for displaying the events in the predetermined period by type and in association with the monitored person Ob and a region in which the events detected by the sensor device SU are counted for each person or each type and displayed for each of the plurality of monitored persons Ob in the personal analysis display screen 63. More specifically, each counting result (each first counting result) by type obtained by counting the events corresponding to the monitored person Ob in the predetermined period by type is displayed in each field (each row or column) provided for each monitored person Ob by each of the bar graphs arranged in order of the type of the event (bar graph in each column or row for each event), and the comparison result by type which changes by equal to or more than the predetermined threshold on the basis of the analysis result and the comparison result by type which does not change by equal to or more than the predetermined threshold on the basis of the analysis result are displayed in the display modes different from each other. In the example illustrated in Fig. 16, to display the counting results regarding the plurality of monitored persons Ob, the personal analysis display region 631 includes first to seventh graph display regions 631a1 to 631a7 for seven people in the rows arranged from the upper side to the lower side. Then, to display each counting result by type for one person by a bar graph, each of the first to seventh graph display regions 631a1 to 631a7 further includes sub-graph regions 631b as many as the number of types of the events. In the present embodiment, the sub-graph region 631b includes a get-into-bed sub-graph region 631b1 which indicates a counting result of the number of times to get into the bed for one person by a bar graph, a get-up sub-graph region 631b2 which indicates a counting result of the number of times to get up for one person by a bar graph, a leave-from-bed sub-graph region 631b3 which indicates a counting result of the number of times to leave the bed for one person by a bar graph, a fall-down sub-graph region 631b4 which indicates a counting result of the number of times to fall down for one person by a bar graph, a breathing abnormality sub-graph region 631b5 which indicates a counting result of the number of times of breathing abnormality for one person by a bar graph, and a nurse call sub-graph region 631b6 which indicates a counting result of the number of times of nurse calls for one person by a bar graph. For example, in the first graph display region 631a1 which is the first region from the top, in the get-into-bed sub-graph region 631b1, the get-up sub-graph region 631b2, the leave-from-bed sub-graph region 631b3, the fall-down sub-graph region 631b4, the breathing abnormality sub-graph region 631b5, and the nurse call sub-graph region 631b6, a bar graph indicating once, a bar graph indicating five times, a bar graph indicating three times, a bar graph indicating twice, a bar graph indicating three times, and a bar graph indicating three times are respectively displayed. The bar graphs displayed in the personal analysis display region 631 are displayed in the display modes different from each other according to the comparison result by type which changes by equal to or more than the predetermined threshold on the basis of the analysis result and the comparison result by type which does not change by equal to or more than the predetermined threshold on the basis of the analysis result. For example, in a case where the comparison result by type changes by equal to or more than the predetermined threshold, the bar graph is indicated by hatching of a plurality of points, and in a case where the comparison result by type does not change by equal to or more than the predetermined threshold, the bar graph is indicated by an outline. In the example illustrated in Fig. 16, the bar graph displayed in the nurse call sub-graph region 631b2 in the sixth graph display region 631a6 which is the sixth region from the top is hatched by a plurality of points, and this indicates that the comparison result by type changes by equal to or more than the predetermined threshold. In a case where the number of people to be displayed exceeds the number of people who can be displayed in the personal analysis display region 631 (631a and 631b), the personal analysis display region 631 (631a and 631b) can be scrolled upward and downward.

For example, in the time chart personal history display screen 61 illustrated in Fig. 13, in the graph personal history display screen 62 illustrated in Fig. 14, and in the personal analysis display screen 63 illustrated in Fig. 16, when the surveillant (user) performs an input operation on the "personal analysis" button 6162, performs an input operation on the "graph" button 6131, performs an input operation on the monitored-person menu region 612a, performs input operations on the period input region 614 and the period length menu region 615, and performs an input operation on the "transmission" button 610, the terminal devices SP and TA (here, fixed terminal device SP) transmit the personal analysis request communication signal, which stores the second instruction for requesting personal analysis of the event detected by the sensor device SU, the monitored person's name of each of the plurality of monitored persons Ob input and designated in the monitored-person menu region 612a (all people collectively designated information in example illustrated in Fig. 16), the periods input and designated in the period input region 614 and the period length menu region 615 (one day on May 14, 2016 in example in Fig. 16), and the graph display mode, to the management server device SV.

When receiving the personal analysis request communication signal, the management server device SV sequentially executes the processing S21, S22, and S25, and in the processing S25, the management server device SV executes the processing S251, S252, and S253. In the processing S253, the monitoring processing part 222 generates an electronic file of the personal analysis display screen 63 illustrated in Fig. 16 by using the predetermined markup language on the basis of the extraction result of the history information extraction processing part 223 by the processing S251 and the analysis result of the analysis processing part 224 by the processing S252.

Returning to Fig. 12, next, the control processing unit 22 replies the analysis information notification communication signal which stores the electronic file of the personal analysis display screen 63 generated in the processing S253 to the terminal devices SP and TA (here, fixed terminal device SP) by the monitoring processing part 222 and terminates the present processing S25. When receiving the analysis information notification communication signal, the terminal devices SP and TA (here, fixed terminal device SP) display (update) the personal analysis display screen 63 by using the electronic file of the personal analysis display screen 63 stored in the received analysis information notification communication signal.

In the processing S25, the management server device SV processes the personal analysis request communication signal received from the terminal devices SP and TA in the processing S21.

Returning to Fig. 9, in the processing S26, the management server device SV executes appropriate processing according to the communication signal received in the processing S21 and executes the following processing S27 by the SV control processing unit 22.

Then, in the processing S27 which is executed after the processing S23 to S26, the management server device SV determines whether to terminate (stop) the operation by the SV control processing unit 22. According to the result of the determination, in a case where the operation is terminated (stopped) (Yes), the management server device SV terminates the present processing, and in a case where the operation is not terminated (stopped) (No), the management server device SV returns the procedure to the processing S21.

Regarding the first event notification communication signal, the personal history request communication signal, the personal analysis request communication signal, and the like, the management server device SV operates as described above.

As described above, since the monitored-person monitoring system MS according to the present embodiment, the management server device SV as an example of the central processing device, and the central processing method implemented in the same make the predetermined display device, for example, the terminal devices SP and TA display the events in the predetermined period by type and in association with the monitored person Ob, the surveillant can visually grasp the detection result of the event regarding the monitored person Ob by type by referring to the display and can easily find tendency of each event. For example, the surveillant can find characteristics of individuals such as the monitored person Ob who issues nurse calls unnecessarily. Therefore, the monitored-person monitoring system MS, the central processing device (management server device SV), and the central processing method can assist in finding the tendency in the notification (information) issued by the monitored person Ob.

Since the monitored-person monitoring system MS, the central processing device (management server device SV), and the central processing method make the display device, for example, the terminal devices SP and TA, display the detected events regarding the monitored person Ob by type with marks on the time axis, the surveillant can easily find the temporal tendency of the detected events regarding the monitored person Ob by type by referring to the display.

Since the monitored-person monitoring system MS, the central processing device (management server device SV), and the central processing method make the display device such as the terminal devices SP and TA display whether the notification has been issued, the surveillant can visually recognize whether the notification has been issued by referring to the display.

Since the monitored-person monitoring system MS, the central processing device (management server device SV), and the central processing method make the display device, for example, the terminal devices SP and TA display the sleeping time and the lying time excluding the sleeping time in display modes different from each other, the surveillant can visually recognize a so-called normal sleeping time at night (night sleeping time) and a so-called daytime nap time (daytime sleeping time) by referring to the display.

Since the monitored-person monitoring system MS, the central processing device (management server device SV), and the central processing method make the display device, for example, the terminal devices SP and TA display the comparison result by type which changes by equal to or more than the predetermined threshold, the surveillant can recognize the type of the event of which the tendency of the number of detections changes between the second period to be analyzed and the third period before the second period.

In the graph display mode of the personal history display mode, the monitored-person monitoring system MS, the central processing device (management server device SV), and the central processing method can recognize the type of the event of which the tendency of the number of detections changes for each of the plurality of sub-periods regarding the single monitored person Ob.

In the personal analysis display mode, the monitored-person monitoring system MS, the central processing device (management server device SV), and the central processing method can recognize the type of the event of which the tendency of the number of detections changes for each of the plurality of monitored persons Ob.

In the above embodiment, the sleeping time (night sleeping time) maybe used as an index (sleeping amount index) indicating a degree of a sleeping amount. For example, the sleeping amount index is a percentage obtained by dividing the sleeping time by a preset nighttime in 24 hours ((sleeping amount index) = {(sleeping time)/(nighttime)} × 100[%]). Such a sleeping amount index is displayed, for example, in the time chart personal history display screen 61. For example, the sleeping amount index is written in a bar 6191 indicating the night sleeping time.

Furthermore, in the above embodiment, the lying time excluding the sleeping time (daytime sleeping time) may be used as an index (activity amount index) indicating a degree of an activity amount during the day. For example, the activity amount index is a percentage obtained by dividing the subtraction result, which is obtained by subtracting the sleeping time excluding the sleeping time from the daytime, by the preset daytime in 24 hours ((activity amount index) = [{(daytime) - (lying time excluding sleeping time)}/(daytime)] × 100 [%], (nighttime) + (daytime) = 24 [hours]). Such an activity amount index is displayed, for example, in the time chart personal history display screen 61. For example, the activity amount index is written in a bar 6192 indicating the daytime sleeping time. In this case, since the activity amount index is 100% in a case of no daytime sleeping time, the display of the activity amount index is omitted.

Although techniques in various modes are disclosed herein, main techniques will be summarized below.

A central processing device according to one aspect is the central processing device for a monitored-person monitoring system for monitoring a monitored person which includes a sensor device which is provided in correspondence with a monitored person to be monitored and detects a predetermined event related to the monitored person, a central processing device which is communicably connected to the sensor device and manages the event which has been detected by and received from the sensor device, and a terminal device which is communicably connected to the central processing device and receives a notification of the event detected by the sensor device via the central processing device. The central processing device includes a storage unit, a history information extraction processing part, and a monitoring processing part. The storage unit stores the event which has been detected by and received from the sensor device as monitoring information associated with the monitored person corresponding to the sensor device, the monitoring information includes a detection time or a reception time of the event and a type of the event, the history information extraction processing part extracts the type of the event corresponding to a predetermined monitored person within a predetermined period from the monitoring information stored in the storage unit, and the monitoring processing part makes a predetermined display device display the events in the predetermined period by type and in association with the monitored person on the basis of the extraction result of the history information extraction processing part. Preferably, in the central processing device, the predetermined event includes, for example, a predetermined behavior which has been preset such as to get into the bed, to get up, to leave the bed, to fall down, and breathing abnormality. Preferably, in the central processing device, the predetermined event includes a nurse call. Preferably, in the central processing device, the storage unit includes a sensor information storage part which stores a sensor ID which is an identifier to specify and identify the sensor device and a monitored person's name of the monitored person in association with each other as sensor information and a monitoring information storage part which stores the sensor ID, a type name of the event, and the detection time or the reception time in association with each other as the monitoring information, and the storage unit stores the event which has been detected by and received from the sensor device in association with the monitored person corresponding to the sensor device with relational using the sensor ID as monitoring information. Preferably, in the central processing device, the predetermined period includes one day, one week, one month, and three months. Preferably, in the central processing device, the predetermined display device is a display device which is further included in the central processing device. Preferably, in the central processing device, the predetermined display device is a display device (including the terminal device) which is communicably connected to the central processing device, and the monitoring processing part transmits a communication signal which is extracted by the history information extraction processing part in the predetermined period and stores the type of the event and the monitored person's name of the monitored person to the predetermined display device.

Since such a central processing device makes the display device display the events in the predetermined period by type and in association with the monitored person, the surveillant can visually grasp the detection result of the events related to the monitored person by type and easily find the tendency by type. Therefore, the central processing device can assist in finding the tendency in the notification (information) issued by the monitored person.

In another aspect, in the central processing device, the monitoring processing part displays a predetermined mark indicating the type of the event on a time axis which is provided in correspondence with the monitored person and according to the predetermined period at the detection time or the reception time of the event so as to make the predetermined display device display the events in the predetermined period by type and in association with the monitored person on the basis of the extraction result of the history information extraction processing part. Preferably, in the central processing device, the predetermined display device is a display device (including the terminal device) which is communicably connected to the central processing device, and the monitoring processing part stores screen data (electronic file) written in a predetermined markup language, in which the predetermined mark indicating the type of the event is displayed on the time axis which is provided in correspondence with the monitored person and according to the predetermined period at the detection time or the reception time of the event, to the communication signal and transmits the data to the predetermined display device.

Since such a central processing device makes the predetermined display device display the detected event related to the monitored person by type by the mark on the time axis, the surveillant can easily find temporal tendency of the detected event related to the monitored person by type.

In another aspect, in the central processing device, the storage unit further stores reply permission/rejection information indicating whether the event which has been detected by and received from the sensor device is given in notification to the predetermined terminal device by type of the event in association with the monitored person corresponding to the sensor device as notification permission/rejection information, the monitoring processing part executes notification processing for notifying the predetermined terminal device of the event which has been detected by and received from the sensor device on the basis of notification permission/rejection information stored in the storage unit and further stores whether the event has been given in notification by the notification processing in association with the event which has been detected by and received from the sensor device as one piece of the monitoring information, and the monitoring processing part makes the predetermined display device further display whether the event has been given in notification at the time when the events in the predetermined period are displayed on the predetermined display device by type and in association with the monitored person on the basis of the extraction result of the history information extraction processing part.

Since such a central processing device makes the predetermined display device further display whether the event has been given in notification, the surveillant can visually recognize whether the event has been given in notification by referring to the display.

In another aspect, the central processing device further includes an analysis processing part, and the analysis processing part executes analysis processing in which a time from a time to get into the bed to a time to leave the bed on the basis of the extraction result of the history information extraction processing part is obtained as a lying time and a time from a first time to get into the bed on or after a predetermined first time to a first time to leave the bed on or after a predetermined second time on a next day in the obtained lying time is assumed as a sleeping time, the monitoring processing part makes the predetermined display device further display the sleeping time obtained by executing the analysis processing and the lying time excluding the sleeping time in display modes different from each other at the time when the predetermined display device displays the events in the predetermined period by type and in association with the monitored person on the basis of the extraction result of the history information extraction processing part. Preferably, in the central processing device, the sleeping time is used as an index (sleeping amount index) indicating a degree of a sleeping amount. For example, the sleeping amount index is a percentage obtained by dividing the sleeping time by a preset nighttime in 24 hours ((sleeping amount index) = {(sleeping time)/(nighttime)} × 100[%]). Preferably, in the central processing device, the lying time excluding the sleeping time is used as an index (activity amount index) indicating a degree of an activity amount during the day. For example, the activity amount index is a percentage obtained by dividing the subtraction result, which is obtained by subtracting the sleeping time excluding the sleeping time from the daytime, by the preset daytime in 24 hours ((activity amount index) = (daytime) - (lying time excluding sleeping time)}/(daytime)] × 100 [%], (nighttime) + (daytime) = 24 [hours]).

Such a central processing device makes the predetermined display device display the sleeping time and the lying time excluding the sleeping time in display modes different from each other, the surveillant can visually recognize a so-called normal sleeping time at night (night sleeping time) and a so-called daytime nap time (daytime sleeping time) by referring to the display.

In another aspect, in the central processing device, the analysis processing part further executes second analysis processing for obtaining the first counting result by counting the events corresponding to the predetermined monitored person by type within the predetermined second period to be analyzed from the monitoring information stored in the storage unit and for obtaining the second counting result by counting the events corresponding to the predetermined monitored person Ob in a predetermined third period before the second period and obtaining an average value according to the second period length, and comparing the first counting result and the second counting result to obtain the comparison result by type which changes by equal to or more than a predetermined threshold, and the monitoring processing part makes the predetermined display device display the comparison result by type which changes by equal to or more than the predetermined threshold obtained by executing the second analysis processing. Preferably, in the central processing device, each of the second period and the third period includes one day, one week, one month, and three months. Preferably, in the central processing device, the third period is changed according to the second period. Preferably, in the central processing device, the second period and the third period have the same period length.

Since such a central processing device makes the predetermined display device display the comparison result by type which changes by equal to or more than the predetermined threshold, the surveillant can recognize the type of the event of which the tendency of the number of detections changes between the second period to be analyzed and the third period before the second period.

In another aspect, in the above central processing device, the second period includes a plurality of sub-periods, and the analysis processing part executes the second analysis processing in which each of the plurality of sub-periods for each monitored person Ob is assumed as the second period. For example, in a case where the second period is one week, the sub-period is one day. Furthermore, for example, in a case where the second period is one month, the sub-period is one week or one day.

Such a central processing device can recognize the type of the event of which the tendency of the number of detections for each of the plurality of sub-periods for one monitored person.

In another aspect, in the central processing device, the analysis processing part executes the second analysis processing on each of the plurality of monitored persons.

Such a central processing device can recognize the type of the event of which the tendency of the number of detections changes for each of the plurality of monitored persons.

A central processing method according to another aspect is a central processing method for a monitored-person monitoring system to monitor a monitored person which includes a sensor device which is provided in correspondence with the monitored person to be monitored and detects a predetermined event related to the monitored person, a central processing device which is communicably connected to the sensor device and manages the event which has been detected by and received from the sensor device, and a terminal device which is communicably connected to the central processing device and receives a notification of the event detected by the sensor device via the central processing device, the central processing method includes a storage process, a history information extraction process, and a monitoring processing process. In the storage process, the event which has been detected by and received from the sensor device is stored in a storage unit as monitoring information associated with the monitored person corresponding to the sensor device, and the monitoring information includes a detection time or a reception time of the event and a type of the event, in the history information extraction process, a type of the detection result corresponding to a predetermined monitored person within a predetermined period is extracted from the monitoring information stored in the storage unit, and in the monitoring processing process, the events in the predetermined period are displayed on a predetermined display device by type and in association with the monitored person on the basis of the extraction result of the history information extraction process.

Since such a central processing method makes the display device display the events in the predetermined period by type and in association with the monitored person, the surveillant can visually grasp the detection result of the events related to the monitored person by type and easily find the tendency by type. Therefore, the central processing method can assist in finding the tendency in the notification (information) issued by the monitored person.

A monitored-person monitoring system according to another aspect includes a sensor device which is provided in correspondence with a monitored person to be monitored and detects a predetermined event related to the monitored person, a central processing device which is communicably connected to the sensor device and manages the event which has been detected by and received from the sensor device, and a terminal device which is communicably connected to the central processing device and receives a notification of the event detected by the sensor device via the central processing device, and monitors the monitored person, and the central processing device is any one of the central processing devices.

Since such a monitored-person monitoring system uses any one of the central processing devices, the monitored-person monitoring system can assist in finding the tendency of the notification (information) issued by the monitored person.

This application is on the basis of Japanese Patent Application No. 2016-126041 filed on June 24, 2016, the contents of which are included in the present application.

In order to express the present invention, the present invention has been appropriately and sufficiently described through the embodiment with reference to the drawings. However, it should be recognized that those skilled in the art can easily change and/or modify the embodiment. Therefore, as long as variations and modifications made by those skilled in the art are at the level which does not depart from the scope of the claims described in claims, it is understood that the variations and the modifications are included in the scope of the claims.

### Industrial Applicability

According to the present invention, the central processing device for the monitored-person monitoring system, the central processing method, and the monitored-person monitoring system can be provided.

## Claims

1. A central processing device for a monitored-person monitoring system which includes a sensor device which is provided in correspondence with a monitored person to be monitored and detects a predetermined event related to the monitored person, a central processing device which is communicably connected to the sensor device and manages the event which has been detected by and received from the sensor device, and a terminal device which is communicably connected to the central processing device and receives a notification of the event detected by the sensor device via the central processing device and monitors the monitored person, the central processing device comprising:
a storage unit;
a history information extraction processing part; and
a monitoring processing part, wherein
the storage unit stores the event which has been detected by and received from the sensor device as monitoring information associated with the monitored person corresponding to the sensor device,
the monitoring information includes a detection time or a reception time of the event and a type of the event,
the history information extraction processing part extracts a type of an event corresponding to a predetermined monitored person within a predetermined period from the monitoring information stored in the storage unit, and
the monitoring processing part makes a predetermined display device display the events in the predetermined period by type and in association with the monitored person on the basis of the extraction result of the history information extraction processing part.

2. The central processing device according to claim 1, wherein
the monitoring processing part displays a predetermined mark indicating the type of the event on a time axis which is provided in correspondence with the monitored person and according to the predetermined period at the detection time or the reception time of the event so as to make the predetermined display device display the events in the predetermined period by type and in association with the monitored person on the basis of the extraction result of the history information extraction processing part.

3. The central processing device according to claim 2, wherein
the storage unit further stores reply permission/rejection information indicating whether the event which has been detected by and received from the sensor device is given in notification to the predetermined terminal device by type of the event in association with the monitored person corresponding to the sensor device as notification permission information,
the monitoring processing part executes notification processing for notifying the predetermined terminal device of the event which has been detected by and received from the sensor device on the basis of notification permission/rejection information stored in the storage unit and further stores whether the notification has been issued by the notification processing as one piece of the monitoring information in association with the event detected by and received from the sensor device in the storage unit, and
the monitoring processing part makes the predetermined display device further display whether the event has been given in notification at the time when the events in the predetermined period are displayed on the predetermined display device by type and in association with the monitored person on the basis of the extraction result of the history information extraction processing part.

4. The central processing device according to any one of claims 1 to 3, further comprising an analysis processing part, wherein
the analysis processing part executes analysis processing in which a time from a time to get into the bed to a time to leave the bed on the basis of the extraction result of the history information extraction processing part is obtained as a lying time and a time from a first time to get into the bed on or after a predetermined first time to a first time to leave the bed on or after a predetermined second time on next day in the obtained lying time is assumed as a sleeping time, and
the monitoring processing part makes the predetermined display device further display the sleeping time obtained by executing the analysis processing and the lying time excluding the sleeping time in display modes different from each other at the time when the predetermined display device displays the events in the predetermined period by type and in association with the monitored person on the basis of the extraction result of the history information extraction processing part.

5. The central processing device according to any one of claims 1 to 4, wherein
the analysis processing part further executes second analysis processing for obtaining a first counting result by counting the events corresponding to the predetermined monitored person by type within the predetermined second period to be analyzed from the monitoring information stored in the storage unit, for obtaining a second counting result by counting the events corresponding to the predetermined monitored person in a predetermined third period before the second period and obtaining an average value according to a second period length, and comparing the first counting result and the second counting result to obtain the comparison result by type which changes by equal to or more than a predetermined threshold, and
the monitoring processing part makes the predetermined display device display the comparison result by type which changes by equal to or more than the predetermined threshold obtained by executing the second analysis processing.

6. The central processing device according to claim 5, wherein
the second period includes a plurality of sub-periods, and
the analysis processing part executes the second analysis processing in which each of the plurality of sub-periods for each monitored person is assumed as the second period.

7. The central processing device according to claim 5, wherein
the analysis processing part executes the second analysis processing on each of the plurality of monitored persons.

8. A central processing method of a monitored-person monitoring system for monitoring a monitored person which includes a sensor device which is provided in correspondence with the monitored person to be monitored and detects a predetermined event related to the monitored person, a central processing device which is communicably connected to the sensor device and manages the event which has been detected by and received from the sensor device, and a terminal device which is communicably connected to the central processing device and receives a notification of the event detected by the sensor device via the central processing device, the central processing method comprising:
a storage process;
a history information extraction process; and
a monitoring processing process, wherein
in the storage process, the event which has been detected by and received from the sensor device is stored in a storage unit as monitoring information associated with the monitored person corresponding to the sensor device,
the monitoring information includes a detection time or a reception time of the event and a type of the event,
in the history information extraction process, a type of the detection result corresponding to a predetermined monitored person within a predetermined period is extracted from the monitoring information stored in the storage unit, and
in the monitoring processing process, the events in the predetermined period are displayed on a predetermined display device by type and in association with the monitored person on the basis of the extraction result of the history information extraction process.

9. A monitored-person monitoring system for monitoring a monitored person, comprising:
a sensor device that is provided in correspondence with a monitored person to be monitored and detects a predetermined event related to the monitored person;
a central processing device that is communicably connected to the sensor device and manages the event which has been detected by and received from the sensor device; and
a terminal device that is communicably connected to the central processing device and receives a notification of the event detected by the sensor device via the central processing device, wherein
the central processing device is the central processing device according to any one of claims 1 to 7.
